# EUROPEAN PATENT SPECIFICATION

(11) **EP 3 508 620 B1**
(45) Date of publication and mention of the grant of the patent: **19.05.2021**
(21) Application number: 18150785.6
(22) Date of filing: 09.01.2018
(51) Int. Cl.: C25D 7/12, C07C 273/18, C07C 275/10, C07C 275/14, C08G 18/00, C25D 3/38, C07C 211/13, C07C 215/18, C07C 215/50, C07C 217/42, C08G 18/71, C08G 18/73, C08G 18/28, C08G 18/32

(54) **UREYLENE ADDITIVE, ITS USE AND A PREPARATION METHOD THEREFOR**
UREYLENADDITIV, DESSEN VERWENDUNG UND HERSTELLUNGSVERFAHREN DAFÜR
ADDITIF D'URÉYLÈNE, SON UTILISATION ET UN PROCÉDÉ DE PRÉPARATION ASSOCIÉ

(43) Date of publication of application: 10.07.2019
(73) Proprietor: Atotech Deutschland GmbH, 10553 Berlin (DE)
(72) Inventor: Bolton, Dr. Onas, 10553 Berlin (DE); Ackermann, Dr. Stefanie, 10553 Berlin (DE); Adolf, Dr. James, 10553 Berlin (DE); Wu, Dr. Jun, 10553 Berlin (DE); Hoffmann, Nadja, 10553 Berlin (DE); Brunner, Dr. Heiko, 10553 Berlin (DE)
(74) Representative: Atotech Deutschland GmbH

(56) References cited:
- EP-A1- 2 292 679
- EP-A1- 2 735 627
- WO-A1-2011/012475
- GB-A- 1 529 046
- US-A- 5 380 942
- US-A1- 2011 130 391
- US-A1- 2016 076 160

## Description

### Field of the Invention

The present invention concerns ureylene additives, a method for their preparation, a use of said ureylene additives favorably as additive in metal or metal alloy deposition composition, preferably in a copper or copper alloy deposition composition, more preferably as suppressor and/or leveler therein, metal or metal alloy deposition composition comprising said ureylene additive and the use of such composition, and a method for electrolytically depositing a metal or metal alloy layer, preferably a copper or copper alloy layer, onto at least one surface of a substrate.

### Background of the Invention

The wet-chemical deposition of metals and metal alloys (metal plating) is widely used in various industries for the manufacturing of decorative and functional coatings. A large number of metals and metal alloys can thus be formed on various substrates. Of particular interest in the electronics and semiconductor industry is copper plating. Copper is used in these industries for its high conductivity and relatively low price. Copper is used to build up the conductive lines, often by electrolytic copper deposition, this being fast and cost-efficient.

Plating baths for electrolytic deposition of copper are *inter alia* used for manufacturing printed circuit boards and IC substrates where fine structures like trenches, through holes (TH), blind micro vias (BMV) and pillar bumps need to be filled or build up with copper. Another application of such electrolytic deposition of copper is filling of recessed structures such as through silicon vias (TSV) and dual damascene plating or forming redistribution layers (RDL) and pillar bumps in and on semiconducting substrates. Still another application which is becoming more demanding is filling through glass vias, *i.e.* holes and related recessed structures in glass substrates with copper or copper alloys by electroplating.

Conventionally, a combination of various additives is used in such plating bath compositions. For example, electrolytic copper plating baths comprise a multitude of individual additives including levelers, suppressors (also referred to as carriers in the art) and accelerators (also referred to as brighteners in the art).

Also, polymers containing urea groups are long used in this field of the art. Exemplarily, the patent application EP 1 069 211 A2 discloses aqueous copper plating baths comprising a source of copper ions, an acid, a suppressor, an accelerator and a leveler which can be poly[bis(2-chloroethyl)etheralt-1,3-bis[3-(dimethylamino)propyl]urea (CAS-No. 68555-36-2) which contains an organo-bound halide atom (e.g., covalent C-CI bonds) in at least one terminus.

Polymers containing urea groups are further known in the art from EP 2 735 627 A1 as levelers for the electrolytic deposition of copper. Such polymers are can be obtained by a polyaddition of aminourea derivatives and nucleophiles. WO 2011/029781 teaches the same polymers for the electrolytic deposition of zinc.

### Amended Description (clean version) dated 28.11.2019 P60859/EP SH

GB 1 529 046 A refers to substituted or unsubstituted ureido-terminated polyoxyalkylene containing compounds and a method of preparing these compounds.

US 5,380,942 A refers to compounds containing bis ureido groups joined by an alkyleneoxy moiety or recurring alkyleneoxy moieties. The 2-nitrogen of each ureido group can carry groups comprising hydrogen, aryl, alkyl or dialkylaminoalkyl, alike or different.

US 2011/0130391 A1 refers to ureido substituted benzoic acid compounds, compositions comprising the compounds and methods for treating or preventing diseases associated with nonsense mutations of mRNA by administering these compounds or compositions.

US 2009/0205969 A1 describes cross-linked polymers made from urea, N,N-dialkylaminoalkylamine and N,N-bis-(aminoalkyl)-alkylamine as additive for electrolytic zinc deposition.

Similar urea-based polymers are also reported in US 4,157,388 wherein all urea moieties are bridged via nitrogen containing alkanediyl, *i.e.* secondary, tertiary amines and the like. Cationic derivatives and their use in electrolytic plating bath are disclosed in the German patent application DE 10 2005 060 030 A1. The individual urea moieties in these polymers are linked by quaternary ammonium derivatives.

Isocyanates have been long used in the preparative chemistry, in particular in polymer chemistry when e.g. polyurethanes are formed. Typically, molecules with two or more isocyanate moieties are used in these cases. However, such molecules with two or more isocyanate moieties lack sufficient selectivity of the individual isocyanate groups, all having a similar reactivity towards nucleophiles such as amines. Reactions requiring a high regioselectivity are thus problematic. However, when using additives, e.g. in plating processes, it is desirable that the additives are uniform in structure as not all regioisomers and side-products may have the same properties for example when used in a plating bath.

Also, polyurethanes have been used in plating baths. For example, EP 2 489 762 A2 describes polyurethanes to be used in zinc plating baths.

However, such additives when used in acidic copper plating baths are not suitable to fulfil the current and future requirements in manufacture of advanced printed circuit boards, IC substrates and metallization of semiconducting and glass substrates. Depending on the circuitry layout, BMVs' in printed circuit boards and IC substrates need to be filled with copper not only conformally but completely. Typical requirements for BMV filling are for example: obtaining a completely filled BMV while depositing no more than 12 to 18 µm of copper onto the neighboring planar substrate areas and at the same time creating a dimple on the outer surface of the filled BMV of no more than 5 µm.

In metallization of semiconducting wafers, TSV filling must lead to a complete and void-free filling with copper while creating no more than 1/5 of via diameter of overplated copper onto the neighboring planar areas. Similar requirements are demanded for filling through glass vias with copper.

### Objective of the present Invention

It is therefore an objective of the present invention to overcome the shortcomings of the prior art. It is therefore a further objective of the present invention to provide a compound suitable to be used in a metal or metal alloy deposition composition, preferably in a copper or copper alloy deposition composition, e.g. as leveler and/or suppressor. It is a further objective of the present invention to provide a method for the preparation of such compounds. It is yet a further objective of the present invention to provide a metal or metal alloy deposition composition, preferably a copper or copper alloy deposition composition, which ideally is capable of filling recessed structures without voids and/or dimples.

### Summary of the Invention

Above-captioned objectives are solved by the ureylene additive comprising at least one structural unit represented by formula (I) 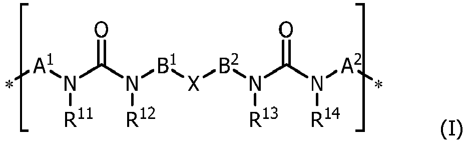 wherein
A¹ and A² are independently selected from the group consisting of oligo(oxyalkanediyl) group and poly(oxyalkanediyl) group;
B¹ and B² are independently alkanediyl groups;
R¹¹, R¹², R¹³ and R¹⁴ are independently selected from the group consisting of hydrogen, alkyl group and aryl group; and
X is selected from the group consisting of
   - structural units represented by formula (II) 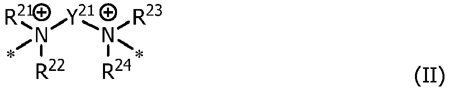 wherein
      R²¹, R²², R²³ and R²⁴ are independently selected from the group consisting of alkyl group and aryl group; and
      Y²¹ is selected from the group consisting of alkanediyl group, alkanediyloxyalkanediyl group, alkanediyloligo(oxyalkanediyl) group and alkanediylpoly(oxyalkanediyl) group;
   - structural units represented by formula (III) 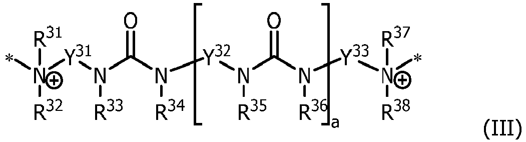 wherein
      R³¹, R³², R³⁷ and R³⁸ are independently selected from the group consisting of alkyl group and aryl group;
      R³³, R³⁴, each R³⁵ and each R³⁶ are independently selected from the group consisting of hydrogen, alkyl group and aryl group;
      Y³¹ and Y³³ are independently alkanediyl groups;
      each Y³² is independently selected from the group consisting of alkanediyl group, alkanediyloxyalkanediyl group, alkanediyloligo(oxyalkanediyl) group and alkanediylpoly(oxyalkanediyl) group; and
      a is an integer selected from the group consisting of 0, 1, 2 and 3;
   - structural units represented by formula (IV) 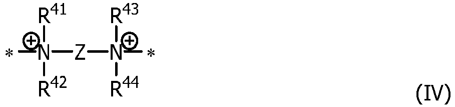 wherein
      R⁴¹, R⁴², R⁴³ and R⁴⁴ are independently selected from the group consisting of alkyl group and aryl group;
      Z is represented by one of the following formulae (Z1) to (Z3) 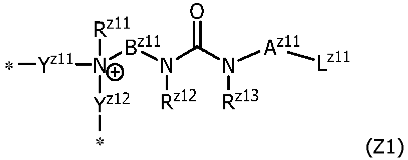
      wherein
      Y^{z11} and Y^{z12} are independently alkanediyl groups;
      A^{z11} is independently selected from the group consisting of oligo(oxyalkanediyl) group and poly(oxyalkanediyl) group;
      B^{z11} is independently selected to be an alkanediyl group;
      R^{z11} is selected from the group consisting of alkyl group and aryl group;
      R^{z12} and R^{z13} are independently selected from the group consisting of hydrogen, alkyl group and aryl group;
      L^{z11} is selected from the group consisting of hydrogen, alkyl group and aryl group; 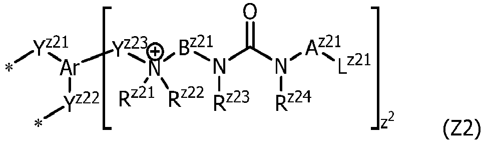
      wherein
      Ar is an aromatic group;
      Y^{z21}, Y^{z22} and each Y^{z23} independently are alkanediyl groups;
      each A^{z21} is independently selected from the group consisting of oligo(oxyalkanediyl) group and poly(oxyalkanediyl) group;
      each B^{z21} is independently selected to be an alkanediyl group;
      each R^{z21} and each R^{z22} are independently selected from the group consisting of alkyl group and aryl group;
      each R^{z23} and each R^{z24} are independently selected from the group consisting of hydrogen, alkyl group and aryl group;
      each L^{z21} is independently selected from the group consisting of hydrogen, alkyl group and aryl group;
      z² is an integer selected from 1 and 2;
      and 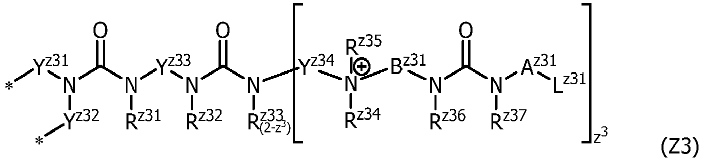
      wherein
      Y^{z31}, Y^{z32}, Y^{z33} and each Y^{z34} are independently alkanediyl groups;
      each A^{z31} is independently selected from the group consisting of oligo(oxyalkanediyl) group and poly(oxyalkanediyl) group;
      each B^{z31} is independently selected to be an alkanediyl group;
      R^{z31}, R^{z32}, R^{z33}, each R^{Z36} and each R^{z37} are independently selected from the group consisting of hydrogen, alkyl group and aryl group;
      each R^{z34} and each R^{z35} are independently selected from the group consisting of alkyl group and aryl group;
      each L^{z31} is independently selected from the group consisting of hydrogen, alkyl group and aryl group;
      z³ is an integer selected from 1 and 2;
   - structural units represented by formula (V) 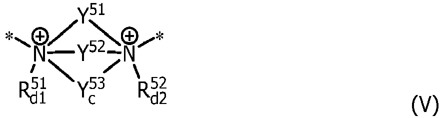 wherein
      each R⁵¹ and each R⁵² are independently selected from the group consisting of alkyl group and aryl group;
      each of Y⁵¹, Y⁵² and Y⁵³ is independently an alkanediyl group;
      c is selected from 0 and 1;
      d1 is an integer expressed by the mathematical equation d1=(1-c);
      d2 is an integer expressed by the mathematical equation d2=(1-c); and
      and
   - structural units represented by formula (VI) 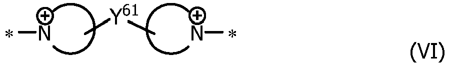 wherein
      Y⁶¹ is selected from the group consisting of alkanediyl group and alkanediyl-urediyl-alkanediyl group.

Above described objectives are further solved by the use of the ureylene additives according to the invention in a metal or metal alloy deposition composition, preferably in a copper or copper alloy deposition composition, more preferably in an electrolytic copper or copper alloy deposition composition, in particular as leveler and/or suppressor therein.

Above described objectives are also solved by the metal or metal alloy deposition composition comprising at least one type of reducible metal ions, preferably copper ions, and at least one ureylene additive according to the invention.

Moreover, above described objectives are solved by the use of the deposition composition according to the invention to form a metal or metal alloy layer, preferably a copper or copper alloy layer, on at least one surface of a substrate.

Further, above described objectives are solved by the method for (preferably electrolytically) depositing a metal or metal alloy layer, preferably a copper or copper alloy layer, onto at least one surface of a substrate comprising the following method steps
(i) providing the substrate having said at least one surface;
(ii) contacting said at least one surface of the substrate with the deposition composition according to the invention comprising the at least one ureylene additive;
(iii) optionally, applying an electrical current between the substrate and at least one anode; and
thereby forming the metal or metal alloy layer, preferably the copper or copper alloy layer, on said at least one surface of the substrate.

Advantageously, the ureylene additives allow for enhanced plating rates to be obtained when used as additives such as suppressors and/or levelers, e.g. in a metal or metal alloy deposition composition such as in an electrolytic copper plating baths (see plating experiments 5.1 versus C2).

### Brief description of the Figures

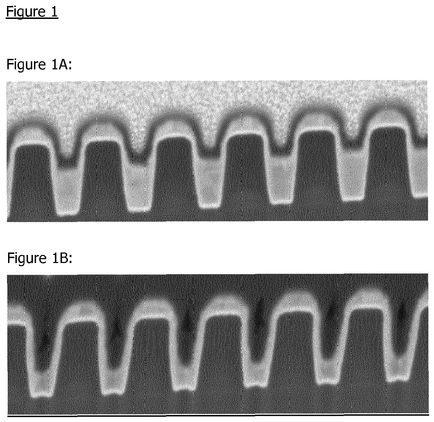

Figure 1 shows the results of the inventive plating experiment 5.1 (Figure 1A) and a comparative plating experiment with a polyethylene imine as leveler (Comparative plating experiment C2 is shown in Figure 1B). Although run under identical conditions otherwise, the filling achieved by the inventive plating experiments is much more complete compared to the comparative plating experiment. It is thus obvious that the present invention allows for improved plating rates which are highly desired in the semiconductor and electronics industry.

### Detailed Description of the Invention

Percentages throughout this specification are weight-percentages (wt.-%) unless stated otherwise. Concentrations given in this specification refer to the volume or mass of the entire solutions / compositions unless stated otherwise. The terms "deposition", "depositing" and "plating" are used interchangeably herein. Also, "layer" and "deposit" are also used synonymously in this specification. It is understood that embodiments of the present invention described in this specification can be combined unless this is technically not feasible or specifically excluded. The terms "deposition composition" and "plating bath" are synonymous in the context of the present invention. The terms "substitution" and "functionalization" are used interchangeably in this specification and in the claims. An "electrolytic metal or metal alloy deposition composition" is herein understood as a composition suitable to be used for electrolytic deposition of a metal or a metal alloy. The isocyanate, the monoamine, the intermediate and the multifunctional starting material are referred to as "reactants" herein.

The term "alkyl group" according to the present invention comprises branched or unbranched alkyl groups comprising cyclic and/or non-cyclic structural elements, wherein cyclic structural elements of the alkyl groups naturally require at least three carbon atoms. C1-CX-alkyl group in this specification and in the claims refers to alkyl groups having 1 to X carbon atoms (X being an integer). C1-C8-alkyl group for example includes, among others, methyl, ethyl, n-propyl, iso-propyl, n-butyl, iso-butyl, sec-butyl, tert-butyl, n-pentyl, iso-pentyl, sec-pentyl, tert-pentyl, neo-pentyl, hexyl, heptyl and octyl. Substituted alkyl groups may theoretically be obtained by replacing at least one hydrogen by a functional group. Unless stated otherwise, alkyl groups are preferably selected from substituted or unsubstituted C1-C8-alkyl groups, more preferably from substituted or unsubstituted C1-C4-alkyl groups because of their improved solubility in water.

The term "aryl group" according to the present invention refers to ring-shaped aromatic hydrocarbon residues, for example phenyl or naphtyl where individual ring carbon atoms can be replaced by N, O and/or S, for example benzothiazolyl. Furthermore, aryl groups are optionally substituted by replacing a hydrogen atom in each case by a functional group. The term C5-CX-aryl group refers to aryl groups having 5 to X carbon atoms (wherein one or more carbon atoms are optionally replaced by N, O and/or S (without changing the number of 5 to X) and X is an integer) in the ring-shaped aromatic group. Unless stated otherwise, aryl group are preferably selected from substituted or unsubstituted C5-C10-aryl groups, more preferably from substituted or unsubstituted C5-C6-aryl groups because of their improved solubility in water. Naturally, a C5-aryl group requires the replacement of at least one carbon atom for a heteroatom capable of donating electrons such as nitrogen, sulfur or oxygen.

The term "alkanediyl group" is the corresponding derivative of an alkyl group having two bonding sites. Sometimes alkanediyl groups are referred to as alkylene groups in the art. C1-CX-alkanediyl group in this specification (X being an integer) and in the claims refers to alkanediyl groups having 1 to X carbon atoms, e.g. 1 - 12. Unsubstituted alkanediyl groups are preferred for the ease of syntheses unless stated otherwise herein. The explanations and preferences outlined for alkyl groups apply to alkanediyl groups as well.

In the art, the term "poly(oxyalkanediyl) group" is often used interchangeably with the terms "polyether", "polyalkylene glycol" and the like. Poly(oxyalkanediyl) groups can be prepared for example by (ring-open) polymerization of suitable epoxides such as alkylene oxides like ethylene oxide, propylene oxide, butylene oxide and/or styrene oxide. An oligo(oxyalkanediyl) group is a shorter derivative of above-named with fewer repeating units derived from above epoxides, e.g. 2 to 4. Poly(oxyalkanediyl) groups usually have 5 or more repeating units.

An alkanediyloxyalkanediyl group can be represented as *-alkanediyl-O-alkanediyl-* . Similarly, an alkanediyloligo(oxyalkanediyl) group and an alkanediylpoly(oxyalkanediyl) group can be represented the same way wherein e.g. the alkanediyl group depicted on the right hand side is replaced by an oligo(oxyalkanediyl) group and a poly(oxyalkanediyl) group, respectively. An alkanediyl-urediyl-alkanediyl group comprises an urediyl group bound between two alkanediyl groups. An urediyl group can be described as di(λ²-azanyl)methanone group (with one R^{ur} bonded to each nitrogen atom). It is optionally represented as 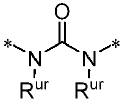 with each R^{ur} being independently selected from the group consisting of hydrogen, alkyl group and aryl group; preferably from the group consisting of hydrogen and C1-C4-alkyl group; they are more preferably hydrogen. Typically, the two alkanediyl groups are bound to the two different nitrogen atoms of the urediyl group. Said alkanediyl-urediyl-alkanediyl group groups can preferably be represented as

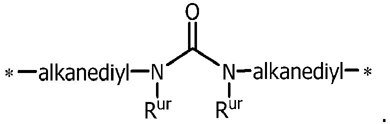

Unless stated otherwise, above-defined groups are substituted or unsubstituted. Functional groups as substituents are preferably selected from the group consisting of oxo (=O), hydroxyl (-OH), amino (-NH₂), carbonyl (-CHO) and carboxyl (-CO₂H) to improve the solubility of the relevant compounds in polar solvents such as water, more preferably hydroxyl to improve the solubility of the relevant compounds in polar solvents such as water and to avoid any potential undesired side-reactions during synthesis. In one embodiment of the present invention, the groups are preferably unsubstituted unless stated otherwise hereinafter. Oxo is not to be mistaken for oxy (-O-) which is usually an oxygen atom of an ether moiety.

If more than one substituent is to be selected from a certain group, each of the substituents is selected independently from each other unless stated otherwise herein. The embodiments described hereinafter can be combined without restraints unless stated otherwise herein or if their combination is technically impossible. Also, the varying numbers of carbon atoms of various preferred embodiments for one residue, e.g. carbon atoms of an alkyl group, can be freely combined. Preferred embodiments described for one aspect of the present invention are applicable *mutatis mutandis* for all the other aspects of the present invention unless stated otherwise herein.

Bonding sites in various chemical formulae are highlighted as asterisks.

If X is a structural unit represented by formula (II), the nitrogen atom bearing R²¹ and R²² is bonded to B¹ and the nitrogen atom bearing R²³ and R²⁴ is bonded to B², respectively.

If X is a structural unit represented by formula (III), the nitrogen atom bearing R³¹ and R³² is bonded to B¹ and the nitrogen atom bearing R³⁷ and R³⁸ is bonded to B², respectively.

If X is a structural unit represented by formula (IV), the nitrogen atom bearing R⁴¹ and R⁴² is bonded to B¹ and the nitrogen atom bearing R⁴³ and R⁴⁴ is bonded to B², respectively.

If Z is represented by formula (Z1), the nitrogen atom bearing R⁴¹ and R⁴² is bonded to Y^{z11} and the nitrogen atom bearing R⁴³ and R⁴⁴ is bonded to Y^{z12}, respectively.

If Z is represented by formula (Z2), the nitrogen atom bearing R⁴¹ and R⁴² is bonded to Y^{z21} and the nitrogen atom bearing R⁴³ and R⁴⁴ is bonded to Y^{z22}, respectively. The individual groups Y^{z21}, Y^{z22} and Y^{z23} are bonded to the ring atoms of the Ar group.

If Z is represented by formula (Z3), the nitrogen atom bearing R⁴¹ and R⁴² is bonded to Y^{z31} and the nitrogen atom bearing R⁴³ and R⁴⁴ is bonded to Y^{z32}, respectively.

In the following and in the dependent claims, further details and preferred embodiments of the urevlene additives which have been found to be particularly useful when applying the ureylene additives as additives in a metal or metal alloy deposition composition, preferably in a copper or copper alloy deposition composition, particularly in an electrolytic copper or copper alloy deposition composition are outlined.

### Formula (I)

Preferably, B¹ and B² are independently a C1-C8-alkanediyl group, more preferably a C2-C6-alkanediyl group, even more preferably a C2-C4-alkanediyl group, yet even more preferably B¹ and B² are independently selected from the group consisting of ethane-1,2-diyl and propane-1,3-diyl, B¹ and B² are most preferably ethane-1,2-diyl.

Preferably, B¹ and B² are unsubstituted alkanediyl groups to avoid side-reactions during synthesis of the ureylene additives.

Preferably, R¹¹, R¹², R¹³ and R¹⁴ are independently selected from the group consisting of hydrogen and C1-C4-alkyl group; more preferably R¹¹, R¹², R¹³ and R¹⁴ are hydrogen.

Preferably, A¹ and A² are independently represented by the following formula (A-1) 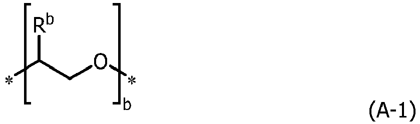 wherein each R^{b} is selected from the group consisting of hydrogen, alkyl group and aryl group; preferably from hydrogen and C1-C4-alkyl group; more preferably from hydrogen and methyl group; b is an integer ranging from 3 to 100, preferably from 5 to 75, more preferably from 7 to 50.

If A¹ and A² are represented by the formula (A-1), they are preferably linked to the respective nitrogen atoms in formula (I) via the carbon atoms bearing R^{b} in said formula.

In one embodiment of the present invention, A¹ and A² are independently poly(oxyalkanediyl) groups. Preferably, said poly(oxyalkanediyl) groups are copolymers. Preferably, the tacticity of the copolymeric poly(oxyalkanediyl) groups is atactic, isotactic or syndiotactic. The poly(oxyalkanediyl) groups can be random copolymers, alternating copolymers or block copolymers. Block copolymers are preferred.

### Formula (II)

Preferably, R²¹, R²², R²³ and R²⁴ are independently C1-C4-alkyl groups; more preferably C1-C2-alkyl groups; most preferably R²¹, R²², R²³ and R²⁴ are methyl groups.

Preferably, Y²¹ is independently selected from the group consisting of C2-C12-alkanediyl group and (C1-C6-alkanediyl)-oxy-(C1-C6-alkanediyl) group, more preferably selected from the group consisting of C3-C8-alkanediyl group and (C2-C4-alkanediyl)-oxy-(C2-C4-alkanediyl) group, even more preferably selected from the group consisting of C3-C6-alkanediyl group and (C2-C3-alkanediyl)-oxy-(C2-C3-alkanediyl) group.

### Formula (III)

The preferences and details given for R¹¹, R¹², R¹³ and R¹⁴ apply independently thereof to each of R³³, R³⁴, each of R³⁵ and R³⁶ and those given for R²¹, R²², R²³ and R²⁴ apply independently thereof to R³¹, R³², R³⁷ and R³⁸.

Preferably, Y³¹ and Y³³ are independently C1-C8-alkanediyl groups, more preferably C2-C6-alkanediyl groups, even more preferably C2-C4-alkanediyl groups.

Preferably, each Y³² is a C2-C12-alkanediyl group, more preferably a C4-C8-alkanediyl group, even more preferably a C5-C7-alkanediyl group.

Preferably, a is 1 or 2, a is more preferably 1.

### Formula (IV)

In one embodiment of the present invention, Z is represented by formula (Z1). In another embodiment of the present invention, Z is represented by formula (Z2). In a further embodiment of the present invention, Z is represented by formula (Z3).

The preferences and details given for R²¹, R²², R²³ and R²⁴ apply independently thereof to R⁴¹, R⁴², R⁴³ and R⁴⁴.

Preferably, each of L^{z11}, L^{z21} and L^{z31} are independently selected from the group consisting of hydrogen, alkyl group and aryl group, each of L^{z11}, L^{z21} and L^{z31} are more preferably independently alkyl groups, even more preferably C1-C4-alkyl groups, yet even more preferably C1-C2-alkyl groups and still even more preferably each of L^{z11}, L^{z21} and L^{z31} are methyl groups.

### Formula (Z1)

The preferences and details outlined for B¹ and B² apply independently thereof *mutatis mutandis to* B^{z11} and those described for A¹ and A² apply independently thereof *mutatis mutandis* to A^{z11}. Further, the preferences and details given for R¹¹, R¹², R¹³ and R¹⁴ apply *mutatis mutandis* thereof to R^{z12} and R^{z13} and those given for R²¹, R²², R²³ and R²⁴ apply *mutatis mutandis* thereof to R^{z11}. Preferably, Y^{z11} and Y^{z12} are independently C1-C6-alkanediyl groups, more preferably C1-C4-alkanediyl group, even more preferably C2-C3-alkanediyl groups.

L^{z11} is preferably an alkyl group, more preferably a C1-C4-alkyl group, even more preferably a C1-C2-alkyl group, still even more preferably a methyl group.

### Formula (Z2)

The preferences and details outlined for B¹ and B² apply independently thereof *mutatis mutandis* for B^{z21} and those described for A¹ and A² apply independently thereof *mutatis mutandis* to A^{z21}. Further, the preferences and details given for R¹¹, R¹², R¹³ and R¹⁴ apply independently thereof to each of R^{z23} and R^{z24} and those given for R²¹, R²², R²³ and R²⁴ apply independently thereof to each of R^{z21} and R^{z22}.

Ar is an aromatic group having at least (z²+2) groups attached thereto and is optionally functionalized, e.g. by one or more hydroxy groups.

Ar is preferably a C5-C6-aromatic group, more preferably a benzene group, even more preferably a hydroxy-substituted benzene group.

Preferably, Y^{z21}, Y^{z22} and Y^{z23} are independently C1-C6-alkanediyl groups, preferably C1-C4-alkanediyl groups, more preferably C1-C2-alkanediyl groups, even more preferably methane-1,1-diyl groups.

Preferably, z² is 1.

L^{z21} is preferably an alkyl group, more preferably a C1-C4-alkyl group, even more preferably a C1-C2-alkyl group, still even more preferably a methyl group.

### Formula (Z3)

The preferences and details outlined for B¹ and B² apply independently thereof *mutatis mutandis* for B^{z31} and those described for A¹ and A² apply independently thereof *mutatis mutandis* to A^{z31}. Further, the preferences and details given for R¹¹, R¹², R¹³ and R¹⁴ apply independently thereof to each of R^{z31}, R^{z32}, R^{z33}, each of R^{z36} and each of R^{z37} and those given for R²¹, R²², R²³ and R²⁴ apply independently thereof to each of R^{z34} and R^{z35}.

Preferably, Y^{z31}, Y^{z32} and each Y^{z34} are independently C1-C8-alkanediyl groups, more preferably C2-C6-alkanediyl groups, even more preferably C3-C4-alkanediyl groups.

Preferably, Y^{z33} is a C1-C12-alkanediyl group, more preferably a C2-C10-alkanediyl group, even more preferably a C4-C8-alkanediyl group, yet even more preferably a C5-C6-alkanediyl group.

L^{z31} is preferably an alkyl group, more preferably a C1-C4-alkyl group, even more preferably a C1-C2-alkyl group, still even more preferably a methyl group.

In formula (Z3), R^{z33} is only present in cases where z³ is 1. Preferably, z³ is 2. In the latter cases, the respective moiety is represented by formula (Z3-1):

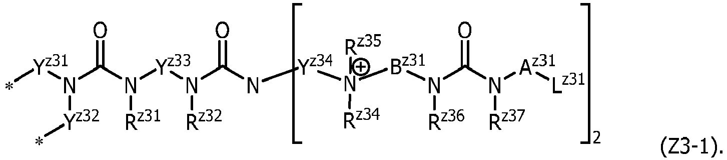

### Formula (V)

The preferences and details given for R²¹, R²², R²³ and R²⁴ apply independently thereof to R⁵¹ and R⁵².

Preferably, each Y⁵¹, Y⁵² and Y⁵³ are independently C1-C6-alkanediyl groups, more preferably C2-C4-alkanediyl groups.

Preferably, c is 1.

In one embodiment of the present invention, the structural unit represented by formula (V) is represented by one of formulae (V-1) or (V-2) 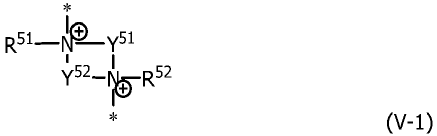 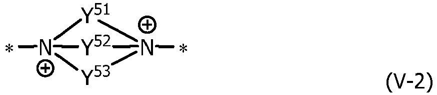 wherein R⁵¹, R⁵²,Y⁵¹, Y⁵² and Y⁵³ are independently selected from above-defined groups. Preferably, the structural unit represented by formula (V) is represented by formula (V-2).

### Formula (VI)

In formula (VI), each 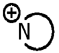 independently represents a charged heterocyclic moiety such as pyridinium (the cationic derivative of pyridine) or pyrrolium (the cationic derivative of pyrrole). These charged heterocyclic moieties naturally include the depicted nitrogen atom(s). The two charged heterocyclic moieties shown in formula (VI) are linked via Y⁶¹.

Preferably, Y⁶¹ is selected from the group consisting of C1-C6-alkanediyl group and C1-C6-alkanediyl-urediyl-C1-C6-alkanediyl group, more preferably C2-C4-alkanediyl group and C1-C3-alkanediyl-urediyl-C1-C3-alkanediyl group.

Preferably, the charged heterocyclic moieties (represented as 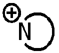 in formula (VI)) are independently selected from the group consisting of pyridinium and pyrrolium, more preferably they are pyridinium.

In one embodiment of the present invention, the structural units represented by formula (VI) are represented by formulae (VI-1) or (VI-2) 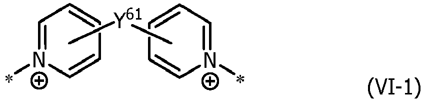 and 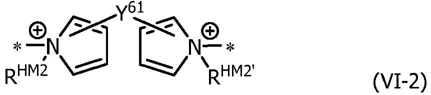 wherein R^{HM2} and R^{HM2'} are independently selected from the group consisting of alkyl group and aryl group, preferably R^{HM2} and R^{HM2'} are independently C1-C4-alkyl groups; more preferably C1-C2-alkyl groups; most preferably methyl groups.

In a preferred embodiment of the present invention, the structural units represented by formula (VI) are represented by formula (VI-1).

In one embodiment of the present invention, X is selected from structural units represented by formulae (II), (III), (IV) and (V). X is preferably selected from structural units represented by formula (II) and structural units represented by formula (III). More preferably, X is a structural unit represented by formula (III). This selection allows for further improvements when using the ureylene additives in a copper deposition composition as the plating rate is increased (compared to other ureylene additives and to standard levelers or suppressors known in the art such as polyethylene imine).

In one embodiment of the present invention, R¹¹, R¹², R¹³, R¹⁴, R³³, R³⁴, R³⁵, R³⁶, R^{z12}, R^{z13}, R^{z23}, R^{z24}, R^{z31}, R^{z32}, R^{z33}, R^{z36} and R^{z37} are independently selected from the group consisting of hydrogen and C1-C4-alkyl group; more preferably they are hydrogen.

In one embodiment of the present invention, R²¹, R²², R²³, R²⁴, R³¹, R³², R³⁷, R³⁸, R⁴¹, R⁴², R⁴³, R⁴⁴, R⁵¹, R⁵², R^{z11}, R^{z21}, R^{z22}, R^{z34} and R^{z35} are independently of each other selected are independently C1-C4-alkyl groups; more preferably C1-C2-alkyl groups; most preferably they are methyl groups. In one embodiment of the present invention, B¹, B², B^{z11}, B^{z21} and B^{z31} are unsubstituted alkanediyl groups for the reasons outlined above.

In one embodiment of the present invention, B¹, B², B^{z11}, B^{z21} and B^{z31} are independently C1-C8-alkanediyl groups, preferably C2-C6-alkanediyl groups, more preferably a C2-C4-alkanediyl groups, they are even more preferably selected from the group consisting of ethane-1,2-diyl and propane-1,3-diyl, they are most preferably ethane-1,2-diyl.

In one embodiment of the present invention, A¹, A², A^{z11}, A^{z21} and A^{z31} are selected from above-defined groups and the preferences and details given for A¹ and A² apply to all of those. Therefore, in one embodiment of the present invention, A¹, A², A^{z11}, A^{z21} and A^{z31} are independently represented by the following formula (A-1) 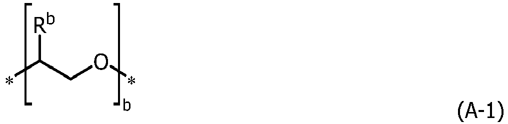 wherein each R^{b} is selected from the group consisting of hydrogen, alkyl group and aryl group; preferably from hydrogen and C1-C4-alkyl group; more preferably from hydrogen and methyl group; b is an integer ranging from 3 to 100, preferably 5 to 75, more preferably 7 to 50.

In a preferred embodiment of the present invention, the ureylene additive according to the invention comprises one (single) structural unit represented by formula (I).

Preferably, the ureylene additive according to the invention does not comprise any halide functional groups (or organobound halides) such as C-CI or C-Br.

Preferably, the ureylene additive according to the invention a capping group L¹¹ linked to A¹ and/or a capping group L¹² linked to A² wherein the capping groups L¹¹ and/or L¹² are independently selected from the group consisting of hydrogen, alkyl group and aryl group; L¹¹ and/or L¹² are preferably independently alkyl groups, more preferably C1-C4-alkyl groups, even more preferably C1-C2-alkyl groups, still even more preferably methyl groups. More preferably, the ureylene additive comprises both capping groups L¹¹ and L¹².

Particularly preferable, the ureylene additive according to the invention comprises one (single) structural unit represented by formula (I) and a capping group L¹¹ linked to A¹ and a capping group L¹² linked to A². This particularly preferred ureylene additive is represented by formula (VII) with the provisos, details and preferences described above applying *mutatis mutandis*:

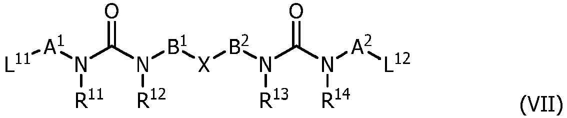

As the ureylene additives according to the invention are cationically charged, they require sufficient anions to be present as counter-charges. Such anions can be hydroxide ions, sulfate ions, hydrogen sulfate ions, carbonate ions, hydrogen carbonate ions, alkylsulfonate ions such as methane sulfonate, alkarylsulfonate ions, arylsulfonate ions such as tosylate, alkylcarboxylate ions, alkaryl-carboxylate ions, arylcarboxylate ions, phosphate ions, hydrogenphosphate ions, dihydrogenphosphate ions, phosphonate ions, and halide ions such as chloride ions. Preferred are halide ions, sulfate ions, alkylsulfonate ions and arylsulfonate ions.

In one embodiment of the present invention, halide ions serving as the counter ions of the positively charged ureylene additive according to the invention are replaced after preparation of the ureylene additive according to the invention by other anions selected from those described above. The halide ions can be for example replaced by ion exchange over a suitable ion exchange resin. The most suitable ion exchange resins are basic ion exchange resins such as Amberlyst® A21. Halide ions can then be replaced by adding an inorganic acid and/or an organic acid containing the desired anions to the ion exchange resin. The enrichment of halide ions in a deposition composition, in particular copper plating bath, during use can be avoided if the ureylene additives according to the invention are free of halide ions.

### Method for preparing the ureylene additive

The present invention further concerns a method for preparing an ureylene additive, preferably the ureylene additive according to the invention. This preparation method includes reacting at least one isocyanate represented by formula (ISO) 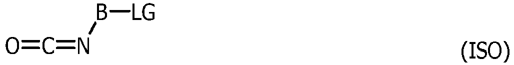 wherein
B is an alkanediyl group; and
LG is a leaving group suitable to be replaced by nucleophilic substitution;
with
at least one monoamine comprising a structural unit represented by formula (MA) 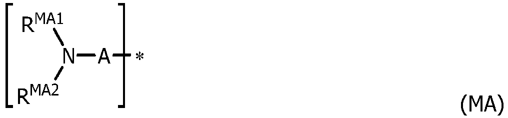 wherein
A is selected from the group consisting of oligo(oxyalkanediyl) group and poly(oxyalkanediyl) group; and
each R^{MA1} and R^{MA2} is selected from the group consisting of hydrogen, alkyl group and aryl group;
and thereby forming at least one intermediate;
and said at least one intermediate is reacted with at least one multifunctional starting material selected from the group consisting of diamines, ureaamines, triamines, aromatic amines, diureaoligoamines, cyclic amines and heterocyclic amines wherein
the diamines are represented by formula (DA) 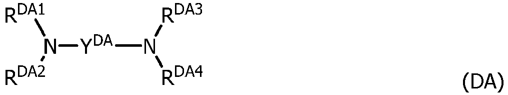 wherein
R^{DA1}, R^{DA2}, R^{DA3} and R^{DA4} are independently selected from the group consisting of alkyl group and aryl group; and
Y^{DA} is selected from the group consisting of alkanediyl group, alkanediyloxyalkanediyl group, alkanediyloligo(oxyalkanediyl) group, alkanediylpoly(oxyalkanediyl) group;
the ureaamines are represented by formula (UA) 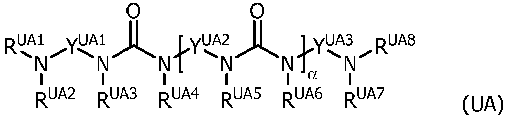 wherein
R^{UA1}, R^{UA2}, R^{UA7} and R^{UA8} are independently selected from the group consisting of alkyl group and aryl group; and
R^{UA3}, R^{UA4}, each R^{UA5} and each R^{UA6} are independently selected from the group consisting of hydrogen, alkyl group and aryl group;
Y^{UA1} and Y^{UA3} are independently alkanediyl groups;
each Y^{UA2} is independently selected from the group consisting of alkanediyl group, alkanediyloxyalkanediyl group, alkanediyloligo(oxyalkanediyl) group and alkanediylpoly(oxyalkanediyl) group; and
α is an integer selected from the group consisting of 0, 1, 2 and 3;
the triamines are represented by formula (TA) 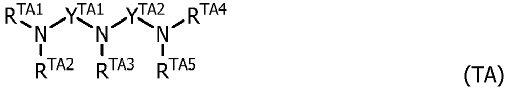 wherein
Y^{TA1} and Y^{TA2} independently are alkanediyl groups;
R^{TA1}, R^{TA2}, R^{TA3}, R^{TA4} and R^{TA5} are independently selected from the group consisting of alkyl group and aryl group;
the aromatic amines are represented by formula (AA) 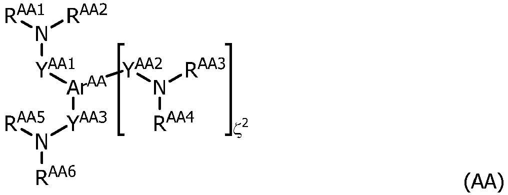 wherein
Ar^{AA} is an aromatic group;
Y^{AA1}, each Y^{AA2} and Y^{AA3} independently are alkanediyl groups;
R^{AA1}, R^{AA2}, R^{AA3}, R^{AA4}, R^{AA5}, R^{AA5} and R^{AA6} are independently selected from the group consisting of alkyl group and aryl group;
ζ² is an integer selected from 1 and 2;
the diureaoligoamines are represented by formula (OA) 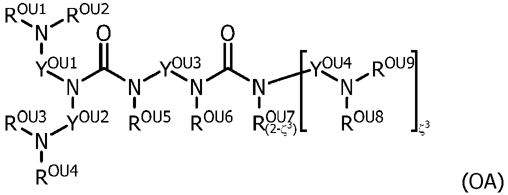 wherein
Y^{OU1}, Y^{OU2}, Y^{OU3} and each Y^{OU4} are independently alkanediyl groups;
R^{OU5}, R^{OU6} and each R^{OU7} are independently selected from the group consisting of hydrogen, alkyl group and aryl group;
R^{OU1}, R^{OU2}, R^{OU3}, R^{OU4}, each R^{OU8}, and each R^{OU9} are independently selected from the group consisting of alkyl group and aryl group;
ζ³ is an integer selected from 1 and 2; and
the cyclic amines are represented by formula (CA) 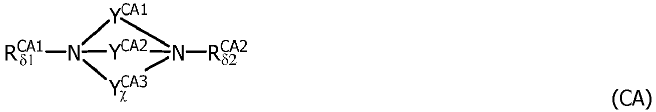 wherein
R^{CA1} and R^{CA2} is independently selected from the group consisting of alkyl group and aryl group;
Y^{CA1}, Y^{CA2} and Y^{CA3} are independently selected from the group consisting of alkanediyl;
χ is an integer selected from 0 and 1; and
δ1 is an integer expressed by the mathematical equation δ1=(1-χ);
δ2 is an integer expressed by the mathematical equation δ2=(1-χ);
the heterocyclic amines are represented by formula (HA) 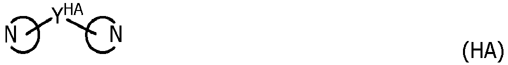 wherein
Y^{HA} is selected from the group consisting of alkanediyl group and alkanediyl-urediyl-alkanediyl group.

Hereinafter, preferred embodiments of the reactants are outlined which were found to be particularly useful, especially when using the resulting ureylene additives in electrolytic copper or copper alloy deposition compositions, e.g. as suprressors and/or levelers.

### Isocyanate (ISO)

Preferably, B is a C1-C8-alkanediyl group, preferably a C2-C6-alkanediyl group, more preferably a C2-C6-alkanediyl group, even more preferably a C2-C4-alkanediyl group, yet even more preferably B is selected from the group consisting of ethane-1,2-diyl and propane-1,3-diyl, most preferably B is ethane-1,2-diyl.

LG is a leaving group suitable to be replaced by nucleophilic substitution such as by an amine group, e.g. by one of the amine groups of the at least one multifunctional starting material. LG is not an isocyanate group. Typical leaving groups for LG are preferably selected from the group consisting of halides such as chloride, bromide, iodide, alkanesulfonate such as mesylate, arylsulfonate such as tosylate, LG is more preferably selected from the group consisting of chloride and bromide, even more preferably chloride. The LG, once replaced by nucleophilic substitution, can form the basis for the anions to be present as counter-charges for the ureylene additive.

In one embodiment of the present invention, the at least one isocyanate is selected from the group consisting of 1-chloro-2-isocyanatoethane (CAS no. 1943-83-5), 1-bromo-2-isocyanatoethane (CAS no. 42865-19-0), 1-chloro-3-isocyanatopropane (CAS no. 13010-19-0), 2-chloroacetyl isocyanate (CAS no. 4461-30-7), methyl 2-isocyanatoethane-1-sulfonate and mixtures of the aforementioned. Preferably, the at least one isocyanate is selected from the group consisting of 1-chloro-2-isocyanatoethane (CAS no. 1943-83-5), 1-chloro-3-isocyanatopropane (CAS no. 13010-19-0), and mixtures of the aforementioned. Most preferably, the at least one isocyanate is 1-chloro-2-isocyanatoethane. The preferences are due to improved yields and the reduced occurrence of side-reactions.

### Monoamine (MA)

The at least one monoamine preferably has a single amine moiety which reacts in the method for preparing the ureylene additive according to the invention with the at least one isocyanate whereby the at least one intermediate is formed. The at least one intermediate comprises an urea moiety which is formed from the amine moiety of the at least one monoamine and the isocyanate moiety of the isocyanate.

In one embodiment of the present invention, A is a poly(oxyalkanediyl) group. Preferably, said poly(oxyalkanediyl) group forming A is a copolymer. Its tacticity is preferably atactic, isotactic or syndiotactic. The poly(oxyalkanediyl) group can be a random copolymer, an alternating copolymer or a block copolymer. A block copolymer is preferred.

It is therefore preferred that the at least one monoamine comprises a structural unit represented by formula (MA-1) 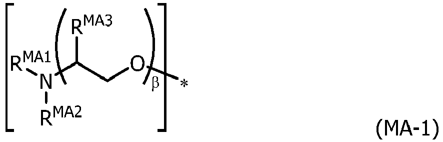 wherein each R^{MA3} is selected from the group consisting of hydrogen, alkyl group and aryl group; preferably from hydrogen and C1-C4-alkyl group; more preferably from hydrogen and methyl group; and β is an integer ranging from 3 to 100, preferably from 5 to 75, more preferably from 7 to 50.

R^{MA1} and R^{MA2} are preferably selected from the group consisting of hydrogen and C1-C4-alkyl group; more preferably, it is hydrogen. It is particularly preferred that at least one of R^{MA1} and R^{MA2} is hydrogen to facilitate the formation of the intermediate.

In one embodiment of the present invention, the at least one monoamine comprises a capping group L linked to A. It is thus preferred that the at least one monoamine is represented by the following formula (MA-2) 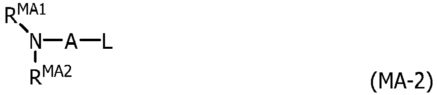 wherein R^{MA1}, R^{MA2} and A are selected from the groups defined above and L is selected from the group consisting of hydrogen, alkyl group and aryl group, preferably alkyl group, more preferably C1-C4-alkyl group, even more preferably C1-C2-alkyl group, still even more preferably methyl group. L is a capping group for A.

In a preferred embodiment of the present invention, the at least one monoamine is represented by the following formula (MA-3) 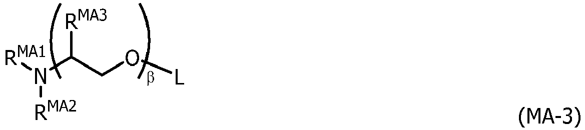 with R^{MA1}, R^{MA2}, R^{MA3}, β and L being selected from above-defined groups.

In one embodiment of the present invention, the at least one monoamine is selected from the group consisting of Surfonamine® B-60 (CH₃-[OCH₂CH₂]₁-[OCH₂CH(CH₃)]₉-NH₂), Surfonamine®B-100 (C₉H₁₉-ϕ-[OCH₂CH(CH₃)]₁₂.₅-OCH₂CH(CH₃)-NH₂), Surfonamine® B-200 (CH₃-[OCH₂CH₂]₆-[OCH₂CH(CH₃)]₂₉-NH₂), Surfonamine® L-100 (CH₃-[OCH₂CH_{2]19}-[OCH2CH(CH3)]₃-NH₂), Surfonamine® L-200 (CH₃-[OCH₂CH₂]₄₁-[OCH₂CH(CH₃)]₄-NH₂), Surfonamine® L-207 (CH₃-[OCH₂CH₂]₃₃-[OCH₂CH(CH₃)]₁₀-NH₂), Surfonamine® L-300 (CH₃-[OCH₂CH₂]₅₈-[OCH2CH(CH₃)]₈-NH₂), Jeffamine® M-600 (CH₃-[OCH₂CH₂]₁-[OCH₂CH(CH₃)]₉-NH₂), Jeffamine® M-1000 (CH₃-[OCH₂CH₂]₁₉-[OCH2CH(CH3)]₃-NH2), Jeffamine® M-2005 (CH₃-[OCH₂CH₂]₆-[OCH₂CH(CH₃)]₂₉-NH₂) and Jeffamine® M-2070 (CH₃-[OCH₂CH₂]₃₁-[OCH₂CH(CH₃)]₁₀-NH₂) and mixtures of the aforementioned. Jeffamines® and Surfonamines® are manufactured and distributed by Huntsman Corp.

Preferably, the at least one monoamine is selected from the group consisting of Surfonamine® B-60 (CH₃-[OCH₂CH₂]₁-[OCH₂CH(CH₃)]₉-NH₂), Surfonamine® B-200 (CH₃-[OCH₂CH₂]₆-[OCH₂CH(CH₃)]₂₉-NH₂), Surfonamine® L-100 (CH₃-[OCH₂CH₂]₁₉-[OCH2CH(CH3)]₃-NH2), Surfonamine® L-200 (CH₃-[OCH₂CH₂]₄₁-[OCH₂CH(CH₃)]₄-NH₂), Surfonamine® L-207 (CH₃-[OCH₂CH₂]₃₃-[OCH₂CH(CH₃)]₁₀-NH₂), Surfonamine® L-300 (CH₃-[OCH₂CH₂]₅₈-[OCH2CH(CH₃)]₈-NH₂), Jeffamine® M-600 (CH₃-[OCH₂CH₂]₁-[OCH₂CH(CH₃)]₉-NH₂), Jeffamine® M-1000 (CH₃-[OCH₂CH₂]₁₉-[OCH2CH(CH3)]₃-NH₂), Jeffamine® M-2005 (CH₃-[OCH₂CH₂]₆-[OCH₂CH(CH₃)]₂₉-NH₂) and Jeffamine® M-2070 (CH₃-[OCH₂CH₂]₃₁-[OCH₂CH(CH₃)]₁₀-NH₂) and mixtures of the aforementioned.

### Multifunctional starting materials

### Diamines (DA)

Preferably, R^{DA1}, R^{DA2}, R^{DA3} and R^{DA4} are independently C1-C4-alkyl groups, more preferably C1-C2-alkyl groups, most preferably R^{DA1}, R^{DA2}, R^{DA3} and R^{DA4} are methyl groups.

Preferably, Y^{DA} is selected from the group consisting of C2-C12-alkanediyl group and (C1-C6-alkanediyl)-oxy-(C1-C6-alkanediyl) group, more preferably selected from the group consisting of C3-C8-alkanediyl group and (C2-C4-alkanediyl)-oxy-(C2-C4-alkanediyl) group, even more preferably selected from the group consisting of C3-C6-alkanediyl group and (C2-C3-alkanediyl)-oxy-(C2-C3-alkanediyl) group.

In one embodiment of the present invention, the at least one diamine is selected from the group consisting of 2,2'-oxybis(N,N-dimethylethan-1-amine) (CAS no. 3033-62-3), N-(2-(dimethylamino)ethyl)-*N¹,N²*,*N²*-trimethylethane-1,2-diamine (CAS no. 3030-47-5), 1,3-bis(dimethylamino)propan-2-ol (CAS no. 5966-51-8), 1,3-di(pyridin-4-yl)propane (CAS no. 17252-51-6) and mixtures of the aforementioned.

### Ureaamines (UA)

The ureaamines comprise two amine moieties which can be used in the reaction with the at least one intermediate.

Preferably, R^{UA1}, R^{UA2}, R^{UA7} and R^{UA8} are independently selected from the group consisting of hydrogen and C1-C4-alkyl group; more preferably they are hydrogen.

Preferably, R^{UA3}, R^{UA4}, each R^{UA5} and each R^{UA6} are independently C1-C4-alkyl groups, more preferably C1-C2-alkyl groups, they are most preferably methyl groups.

Preferably, Y^{UA1} and Y^{UA3} are independently C1-C8-alkanediyl groups, more preferably C2-C6-alkanediyl groups, even more preferably C2-C4-alkanediyl groups.

Preferably, each Y^{UA2} is a C2-C12-alkanediyl group, more preferably a C4-C8-alkanediyl group, even more preferably a C5-C7-alkanediyl group.

Preferably, α is 0, 1 or 2; α is more preferably 0 or 1; even more preferably 0.

In one embodiment of the present invention, the at least one ureaamine is 1,3-bis(3-(dimethylamino)propyl)urea (CAS no. 52338-87-1).

Suitable ureaamines can be exemplarily be prepared by reacting a diisocyanate with a compound having a suitable amino moiety.

### Triamines (TA)

The triamines comprise three amine moieties which can be used in the reaction with the at least one intermediate.

Preferably, Y^{TA1} and Y^{TA2} independently are independently C1-C6-alkanediyl groups, more preferably C1-C4-alkanediyl group, even more preferably C2-C3-alkanediyl groups.

Preferably, R^{TA1}, R^{TA2}, R^{TA3}, R^{TA4} and R^{TA5} are independently selected from the group consisting of C1-C4-alkyl groups; more preferably C1-C2-alkyl groups; they are most preferably methyl groups.

In one embodiment of the present invention, the at least one triamine is a di(alkanediyl)triamine having an alkyl substituent on the central nitrogen atom (*i.e.* the nitrogen atom bearing R^{TA3} in formula (TA)) such as *N¹*-(2-aminoethyl)-*N¹*-methylethane-1,2-diamine) or *N¹*-(2-aminopropyl)-*N¹*-methylpropane-1,3-diamine).

### Aromatic Amines (AA)

The aromatic amines comprise two or more amine moieties which can be used in the reaction with the at least one intermediate.

Ar^{AA} is an aromatic group having at least (ζ²+2) groups attached thereto and is optionally functionalized, e.g. by one or more hydroxy groups.

Preferably, Ar^{AA} is a C5-C6-aromatic group, more preferably a benzene group, even more preferably a hydroxy-substituted benzene group.

Preferably, Y^{AA1}, Y^{AA2} and Y^{AA3} are independently C1-C6-alkanediyl groups, preferably C1-C4-alkanediyl group, more preferably C1-C2-alkanediyl group, even more preferably methane-1,1-diyl group.

Preferably, ζ² is 1.

In one embodiment of the present invention, the at least one aromatic amine is 2,4,6-tris((dimethylamino)methyl)phenol (CAS no. 90-72-2).

### Diureaoligoamines (OA)

The diureaoligoamines comprise three or more amine moieties which can be used in the reaction with the at least one intermediate.

Preferably, Y^{OU1}, Y^{OU2} and each Y^{OU4} are independently C1-C8-alkanediyl groups, more preferably C2-C6-alkanediyl groups, even more preferably C3-C4-alkanediyl groups.

Preferably, Y^{OU3} is a C1-C12-alkanediyl group, more preferably a C2-C10-alkanediyl group, even more preferably a C4-C8-alkanediyl group, yet even more preferably a C5-C6-alkanediyl group. Preferably, R^{OU5}, R^{OU6} and each R^{OU7} are independently selected from the group consisting of hydrogen and C1-C4-alkyl group; they are more preferably hydrogen.

Preferably, R^{OU1}, R^{OU2}, R^{OU3}, R^{OU4}, each R^{OU8}, and each R^{OU9} are independently selected from the group consisting of C1-C4-alkyl groups; more preferably C1-C2-alkyl groups; they are most preferably methyl groups.

Preferably, ζ³ is 2.

In one embodiment of the present invention, the at least one diureaoligoamine is 1-(2-chloroethyl)-3-(1-((1-(2-methoxyethoxy)propan-2-yl)oxy)propan-2-yl)urea.

Cyclic Amines (CA)

Preferably, R^{CA1} and R^{CA2} are independently C1-C4-alkyl groups, more preferably C1-C2-alkyl groups, R^{CA1} and R^{CA2} are most preferably methyl groups.

Preferably, Y^{CA1}, Y^{CA2} and Y^{CA3} are independently is a C1-C6-alkanediyl group, more preferably a C2-C4-alkanediyl group.

Preferably, χ is 1 (and each of δ1 and δ2 are thus 0).

In one embodiment of the present invention, the cyclic amines are represented by one of formulae (CA1) and/or (CA2) 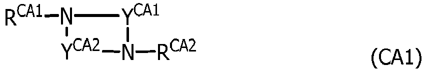 and 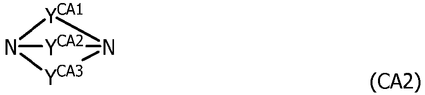 wherein R^{CA1}, R^{CA2} and Y^{CA1}, Y^{CA2} and Y^{CA3} are selected from the groups described above. The cyclic amines represented by formula (CA2) are preferred.

In one embodiment of the present invention, the at least one cyclic amine is 1,4-diazabicyclo[2.2.2]octane (CAS no. 280-57-9).

### Heterocyclic Amines (HA)

Each 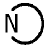 in formula (HA) represents independently a non-charged heterocyclic moiety which are linked via Y^{HA}. The heterocyclic moieties naturally include the depicted nitrogen atom(s).

Preferably, the non-charged heterocyclic moieties are independently selected from the group consisting of pyridine and pyrrole, more preferably they are pyridine.

Preferably, Y^{HA} is selected from the group consisting of C1-C6-alkanediyl group and C1-C6-alkanediyl-urediyl-C1-C6-alkanediyl group, more preferably C2-C4-alkanediyl group and C1-C3-alkanediyl-urediyl-C1-C3-alkanediyl group.

In one embodiment of the present invention, the heterocyclic amines are represented as given in formulae (HA-1) and/or (HA-2) 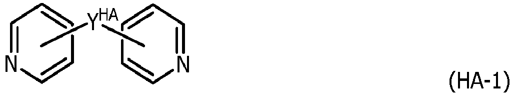 and/or 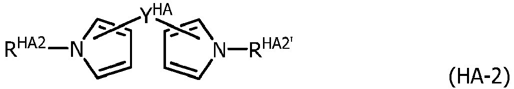 wherein R^{HA2} and R^{HA2'} are independently selected from the group consisting of hydrogen, alkyl group and aryl group, they are preferably independently C1-C4-alkyl groups; more preferably C1-C2-alkyl groups; most preferably methyl groups.

In a preferred embodiment of the present invention, that heterocyclic amines are represented by formula (HA-1).

In one embodiment of the present invention, the at least one heterocyclic amine is 1,3-bis(pyridin-4-ylmethyl)urea (CAS no. 84824-90-8).

Preferably, the at least one multifunctional starting material is a diamine or an ureaamine. More preferably, the at least one multifunctional starting is an ureaamine. This selection allows for a further improvement when using the ureylene additives in a copper deposition composition as the plating rate is increased (compared to other compounds such as conventional levelers or suppressors known in the art such as polyethylene imine).

It is possible to carry out the method for preparing the ureylene additive in two separate reaction steps or as a one-pot-reaction. It is preferred to allow for even higher selectivity to run the reaction in two subsequent reaction steps, *i.e.* the reaction of the at least one isocyanate and the at least one monoamine forming the at least one intermediate (hereinafter referred to as "reaction step I") and then thereafter, the reaction of the at least one intermediate with the at least one multifunctional starting material (hereinafter "reaction step II").

The stoichiometric ratio of the at least one isocyanate to the at least one monoamine in reaction step I preferably ranges from 0.8 to 1.25, more preferably ranges from 0.9 to 1.1 and even more preferably from 0.95 to 1.05. Most preferably, both reactants are used in (substantially) equimolar amounts.

The at least one multifunctional starting material has two or more sites which can suitably be used for reactions with the at least one intermediate formed from the reaction of the at least one isocyanate (hereinafter "reactive sites") and the at least one monoamine. Thus, the stoichiometric ratio of the at least one intermediate to the at least one multifunctional starting material in reaction step II depends on the number of such reactive sites. Preferably, the stoichiometric ratio of the at least one intermediate to each reactive site ranges from 0.8 to 1.25, more preferably ranges from 0.9 to 1.1 and even more preferably from 0.95 to 1.05. Most preferably, the at least one intermediate is used in a (substantially) equimolar amount in relation to the reactive sites of the multifunctional starting material in the preparation of the ureylene additive.

The temperature in reaction step I preferably ranges from 0 to 45 °C, preferably from 5 to 40 °C and more preferably from 10 to 30 °C. The temperature in reaction step II preferably ranges from 50 to 100 °C, preferably from 60 to 95 °C and more preferably from 70 to 90 °C. Temperatures outside above recommendations may be applied depending *inter alia* on the solvent, the reactants and so forth.

The reaction steps can be carried out for any length of time, preferably for a time sufficient to allow for complete conversion of the reactants. The conversion of the reactants can be monitored by standard analytical means known in the art such as chromatography (thin layer chromatography, gas chromatography and the like). The necessary time for this depends *inter alia* on the temperature of the reaction mixtures.

In reaction step I, typical reaction times range from 5 min until the reaction is complete (full conversion of one or all of the reactants), preferably from 15 min to 48 h and more preferably from 30 min to 24 h. In reaction step II, typical reaction times range 1 h until the reaction is complete, preferably from 2 to 240 h and more preferably from 6 to 24 h. Useful reaction times inter alia depend on the reaction conditions used and the reactants employed.

It is preferable to carry out the reactions in at least one solvent. Useful solvents do not result in any side-reactions (of solvent with any of the reactant for example) and allow for homogeneous solutions of the reactants and products of reactions. In the context of the present invention, preferred solvents are non-protic.

Preferably, the at least one solvents to be used in reaction step I is selected from the group consisting of non-protic, non-polar solvents. Non-polar solvents are characterized herein in that the dipole moment is 1.60 Debye or below. More preferably, the at least one solvent to be used in reaction step I is selected from the group consisting of chloroform, dichloromethane, 1,4-dioxane, toluene, benzene and (liquid) alkanes such as cyclohexane, hexane, pentane and mixtures of the aforementioned, it is even more preferably selected from the group consisting of chloroform, dichloromethane and mixtures of the aforementioned because of their superior solubility properties for the reactants and reaction products and their low boiling points making them easy to be removed.

Preferably, the at least one solvents to be used in reaction step II is selected from the group consisting of non-protic, polar solvents. Polar solvents are characterized in that the dipole moment is 1.61 Debye or above. More preferably, the at least one solvent to be used in reaction step II is selected from the group consisting of tetrahydrofuran, ethyl acetate, acetone, dimethylformamide, acetonitrile, dimethyl sulfoxide, nitromethane, propylene carbonate and mixtures of the aforementioned; it is even more preferably selected from the group consisting of ethyl acetate, acetone, acetonitrile and mixtures of the aforementioned because of their superior solubility properties for the reactants and reaction products and their low boiling points making them easy to be removed. The amount of the solvents in reaction step I and reaction step II can be varied greatly and generally, an amount which allows all reactants and the products formed to be dissolved is sufficient. Too high amounts have to be removed after the reactions and are thus to be avoided. It is possible to remove the at least one solvent used after reaction step I and/or reaction step II, e.g. by distillation.

The method for preparing the ureylene additive according to the invention is advantageously very selective and few to none side-reactions occur. This *inter alia* gives rise to excellent yields. Thus, tedious purification steps are not required. However, it is possible to include such a purification step into the method for preparing the ureylene additive according to the invention if desired. Such purification steps are commonly known in the art. Without limitation, they include chromatography, distillation, extraction, flotation or a combination of any of the aforementioned. The purification method to be used depends on the physical properties of the respective compounds present in the reaction mixture and has to be chosen for each individual case.

Advantageously, no catalyst is needed in reaction step I. Catalysts in reaction mixtures are often difficult to remove in technical applications and thus mostly remain in the reaction mixture. As they are typically metallic compounds, they might have detrimental effects, e.g. when such obtained product is used in a deposition composition. The method for preparing the ureylene additive according to the invention is further advantageously very atom-efficient and thus ecological as all atoms of all reactants are included in the final ureylene additive.

In case the method of preparing the ureylene additive is a one-pot-reaction, the solvent is preferably selected from the same group of solvents to be used in reaction step II (including the respective preferred selections). Preferably, the one-pot-reaction includes a first temperature to be used for a first period of time and a second temperature for a second period of time. The first temperature and the first period of time are the same as described for reaction step I and the second temperature and the second period of time are the same as described for reaction step II.

### The Intermediate (INT)

The present invention further concerns an intermediate useful for being reacted with the multifunctional starting material to form the ureylene additive according to the invention.

The intermediate comprises a structural unit represented by formula (INT) 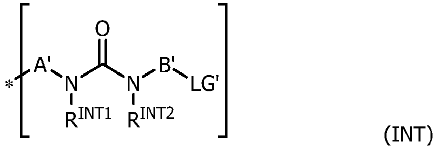 wherein
B' is an alkanediyl group;
LG' is a leaving group suitable to be replaced by nucleophilic substitution;
R^{INT1} and R^{INT2} are independently selected from the group consisting of hydrogen, alkyl group and aryl group;
A' is selected from the group consisting of oligo(oxyalkanediyl) group and poly(oxyalkanediyl) group.

More preferably, the intermediate is represented by formula (INT-1) 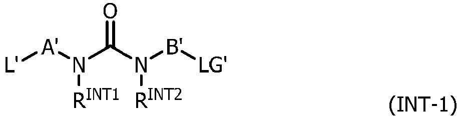 wherein B', LG', R^{INT1}, R^{INT2} and A' are selected from above defined groups (as given in formula (INT) and L' is selected from the group consisting of hydrogen, alkyl group and aryl group.

The preferences and details given for B apply *mutatis mutandis* to B'. The preferences and details given for A apply *mutatis mutandis* to A'. The preferences and details given for LG apply *mutatis mutandis* to LG'. The preferences and details given for R^{MA1} apply *mutatis mutandis* to R^{INT1}. The preferences and details given for R^{MA2} apply *mutatis mutandis* to R^{INT2}. The preferences and details given for L apply *mutatis mutandis* to L'.

The present invention further concerns the reaction of the at least one intermediate and the at least one multifunctional starting material to form the ureylene additive according to the invention.

### Metal or metal alloy deposition composition

The present invention further concerns the use of at least one ureylene additive in a metal or metal alloy deposition composition. The present invention also concerns a metal or metal alloy deposition composition comprising at least one type of reducible metal ions (which are to be deposited), preferably copper ions, and at least one ureylene additive according to the invention.

The metal or metal alloy deposition composition, preferably the copper or copper alloy deposition composition, according to the invention can be used to form a metal or metal alloy layer, preferably a copper or copper alloy layer, on at least one surface of a substrate.

Reducible metal ions are understood in the context of the present invention as those metal ions which can be deposited to form a metal layer or a metal alloy layer (under the given conditions). In the context of the present invention, reducible metal ions are preferably selected from the group consisting of gold ions, tin ions, silver ions, zinc ions, nickel ions, cobalt ions, palladium ions and copper ions. Suitable sources of said ions are water-soluble salts and/or water-soluble complexes of said metals. Non-reducible metal ions include *inter alia* alkaline and earth alkaline metal ions which cannot be reduced under the conditions typically applied.

If only one type of reducible metal ions is present in the deposition composition according to the invention only this metal will be deposited when using the plating bath. If two or more types of reducible metal ions are present therein, an alloy will be deposited. It is also possible within the means of the present invention to deposit an alloy when using certain reducing agents which will form phosphorus or boron based co-deposits such as hypophosphite or aminoboranes with one or more types of reducible metal ions. The deposition compositions according to the invention are suitable for depositing metals or metal alloys which can be used in the electronics and semiconductor industry.

It is possible that two or more ureylene additives are used as a mixture in a deposition composition.

The deposition composition according to the invention is an aqueous solution. The term "aqueous solution" means that the prevailing liquid medium, which is the solvent in the solution, is water. Further liquids, which are miscible with water, as for example alcohols and other polar organic liquids may be added. For its ecologically benign characteristics, it is preferred to use water only (*i.e.* more than 99 wt.-% based on all solvents in the composition, more preferably more than 99.9 wt.-% based on all solvents).

Other components may be added to the deposition composition such as pH adjustors (acids, bases, buffers), complexing agents (also referred to as chelating agents), stabilizing agents, reducing agents, wetting agents and the like. These components and suitable concentration thereof are known in the art.

The deposition composition according to the invention may be prepared by dissolving all components in aqueous liquid medium, preferably in water.

### Copper plating bath

In a preferred embodiment of the present invention, the at least one type of reducible metal ions are copper ions. Such a deposition composition will be herein referred to as "copper plating bath". Said copper plating bath is particularly suitable for electroplating of copper; it is thus preferably an electrolytic copper plating bath. Typically, the inventive copper plating bath is suitable for filling of recessed structures, dual damascene applications and the like.

More preferably, 99 wt.-% or more, even more preferably 99.9 wt.-% or more, of all reducible metal ions in the inventive copper plating bath ions are copper ions. Even more preferably, the inventive copper plating bath is free of further reducible metal ions other than copper ions (disregarding traces of impurities commonly present in technical raw materials and typically employed redox couples such as Fe³⁺/Fe²⁺, *i.e.* less than 0.1 wt.-% of such further reducible metal ions in relation to the copper ions). Particularly, the copper plating bath is free of (intentionally added) zinc ions. Co-deposition of zinc and copper reduces the electrical conductivity of the formed deposits significantly compared to pure copper deposits rendering such co-deposits of zinc and copper less suitable for the use in the electronics industry. Further, it is preferably free of tin ions for the same reasons outlined for zinc ions.

The ureylene additive according to the invention acts as leveler and/or suppressor in the inventive copper plating bath. The leveling function and the term "leveler" means the following: Using the copper plating bath according to the invention and the method according to the invention, it is possible to deposit copper in a very uniform manner in the structures that are to be filled, such as recessions and depressions. In particular, it is possible to fill recessions and depressions totally, reduce a deposition of copper on the surface compared to deposition in the depressions/recessions, and to avoid or at least minimize any voids or dimples. This guarantees that an extensively smooth, even copper surface is formed that exhibits practically no deformations. The suppressing function and the term "suppressors" means that smoother, more homogeneous copper surfaces with fewer voids can be obtained.

The concentration of the at least one ureylene additive according to the invention in the inventive copper plating bath preferably ranges from 1 to 1000 mg/L, more preferably from 5 to 500 mg/L and even more preferably from 50 to 300 mg/L . If more than one ureylene additive is used, the overall concentration of all ureylene additives used is preferably in above-defined ranges.

Copper ions are included in the bath by using at least one water-soluble copper salt or copper complex. Such water-soluble copper salts and copper complexes are preferably selected from the group consisting of copper sulfate, copper alkyl sulfonates such as copper methane sulfonate, copper arylsulfonates such as copper *p*-toluene sulfonate and copper phenyl sulfonate, copper halides such as copper chloride, copper acetate, copper citrate, copper fluoroborate, copper oxide, copper carbonate and mixtures of the aforementioned. More preferably, copper sulfate, copper alkylsulfonates or mixtures of the aforementioned are used as the copper ion source. The copper ion concentration in the copper plating bath preferably ranges from 1 g/L to 70 g/L.

The copper plating bath further contains at least one acid which is preferably selected from the group consisting of sulfuric acid, fluoroboric acid, phosphoric acid and methane sulfonic acid and mixtures of the aforementioned. Said acid is preferably contained in the copper plating bath in a concentration of 1 g/L to 400 g/L, more preferably from 5 g/L to 250 g/L.

The copper plating bath preferably has a pH value of ≤ 3, more preferably ≤ 2, even more preferably of ≤ 1.5, yet even more preferably of ≤ 1.0.

The copper plating bath optionally contains at least one accelerator which is known in the art. Said accelerator is preferably selected from the group consisting of organic thiol-, sulfide-, disulfide- and polysulfide-compounds. More preferred accelerators are selected from the group consisting of 3-(benzthiazolyl-2-thio)-propylsulfonic-acid, 3-mercaptopropan-1-sulfonic acid, ethylendithiodi-propylsulfonic-acid, bis-(p-sulfophenyl)-disulfide, bis-(ω-sulfobutyl)-disulfide, bis-(ω-sulfohydroxypropyl)-disulfide, bis-(ω-sulfopropyl)-disulfide, bis-(ω-sulfopropyl)-sulfide, methyl-(ω-sulfopropyl)-disulfide, methyl-(ω-sulfopropyl)-trisulfide, O-ethyl-dithiocarbonic-acid-S-(ω-sulfopropyl)-ester, thioglycol-acid, thiophosphoric-acid-O-ethyl-bis-(ω-sulfopropyl)-ester, 3-N,N-dimethylaminodithiocarbamoyl-1-propanesulfonic acid, 3,3'-thiobis(1-propanesulfonic acid), thiophosphoric-acid-tris-(ω-sulfopropyl)-ester and their corresponding salts. The concentration of the at least one optional accelerator (or of all if more than one is used) in the copper plating bath preferably ranges from 0.01 mg/L to 100 mg/L, more preferably from 0.05 mg/L to 50 mg/L.

The copper plating bath optionally contains at least one further suppressor which is known in the art. Preferably, the at least one further suppressor is selected from the group consisting of polyvinylalcohol, carboxymethylcellulose, polyethylenglycol, polypropyleneglycol, stearic acid polyglycolester, alkoxylated naphtoles, oleic acid polyglycolester, stearylalcoholpolyglycolether, nonylphenol-polyglycolether, octanolpolyalkyleneglycolether, octanediol-bis-(polyalkyleneglycolether), poly(ethylenglycol-*ran*-propylenglycol), poly(ethylenglycol)-*block*-poly(propylenglycol)-*block-*poly(ethylenglycol), and poly(propyleneglycol)-*block*-poly(ethylenglycol)-*block*-poly(propylenglycol). More preferably, the optional suppressor is selected from the group consisting of polyethylenglycol, polypropylenglycol, poly(ethylenglycol-*ran*-propylenglycol), poly(ethylenglycol)-*block*-poly-(propyleneglycol)-*block*-poly(ethylenglycol) and poly(propyleneglycol)-*block*-poly(ethyleneglycol)-*block*-poly(propylenglycol). The concentration of the at least one optional suppressor (or of all if more than one is used) in the copper plating bath preferably ranges from 0.005 g/L to 20 g/L, more preferably from 0.01 g/L to 5 g/L.

Optionally, the aqueous copper plating bath contains at least one further leveler, which is preferably selected from the group consisting of nitrogen containing organic compounds such as polyethyleneimine, alkoxylated polyethyleneimine, alkoxylated lactams and polymers thereof, diethylenetriamine and hexamethylenetetramine, polyethylenimine bearing peptides, polyethylenimine bearing amino acids, polyvinyl alcohol bearing peptides, polyvinyl alcohol bearing amino acids, polyalkyleneglycol bearing peptides, polyalkyleneglycol bearing amino acids, aminoalkylene bearing pyrroles and aminoalkylene bearing pyridines, organic dyes such as Janus Green B, Bismarck Brown Y and Acid Violet 7, sulfur containing amino acids such as cysteine, phenazinium salts and derivatives thereof. Suitable ureyl polymers have been disclosed in EP 2735627 A1, said polyalkyleneglycol bearing amino acids and peptides are published in EP 2113587 B9 and EP 2537962 A1 teaches suitable aminoalkylene bearing pyrroles and pyridines. The preferred further leveler is selected from nitrogen containing organic pyridinium compounds. Said optional leveler is preferably contained in the copper plating bath in amounts of 0.1 mg/L to 100 mg/L.

The copper plating bath optionally further contains at least one source of halide ions, preferably chloride ions, in a quantity of 20 mg/L to 200 mg/L, more preferably from 30 mg/L to 60 mg/L. Suitable sources for halide ions are for example hydrochloric acid or alkali halides such as sodium chloride. It is also possible that the at least one ureylene additive comprises halide ions which may be used alone or in combination with one or more of above sources.

Optionally, the copper plating bath contains at least one wetting agent. These wetting agents are also referred to as surfactants in the art. The at least one wetting agent may be selected from the group of non-ionic, cationic, anionic and/or amphoteric surfactants and is used in concentration from 0.01 to 5 wt.-%.

In one embodiment of the present invention, a redox couple, such as Fe^{2+/3+} ions is contained in the copper plating bath. Such a redox couple is particularly useful, if reverse pulse plating is used in combination with inert anodes for copper deposition. Suitable processes for copper plating using a redox couple in combination with reverse pulse plating and inert anodes are for example disclosed in US 5,976,341 and US 6,099,711. The copper plating bath is particularly suitable for electrolytic deposition of copper.

In one embodiment of the present invention, a low concentration copper plating bath is used. Said low concentration copper plating bath comprises copper ions in a concentration of 1 to 10 g/L, preferably of 2 to 8 g/L, and an acid in a concentration of 2 to 15 g/L, preferably of 5 to 10 g/L. This increases the overpotential of said bath, making deposition easier to control allowing thus for a more reproducible fill of recessed structures.

In another embodiment of the present invention, a high concentration copper plating bath is used. Said high concentration copper plating bath comprises copper ions in a concentration of 10 to 70 g/L, preferably of 20 to 50 g/L, and an acid in a concentration of 10 to 400 g/L, preferably of 50 to 200 g/L. This increases the conductivity of the bath resulting in a faster copper deposition.

It was found by the inventors that the ureylene additive have a particular positive effect on the plating rate allowing for faster deposition of copper compared to other commonly used levelers such as polyethylene imine.

### Method according to the invention

The steps are preferably carried out in the given order but not necessarily in immediate order. Steps (ii) and optional step (iii) may be carried out at the same time, subsequently or step (ii) is carried out followed by optional step (iii) while step (ii) is continued. It is preferred that the method according to the invention comprises optional step (iii), thus making the metal deposition an electrolytic metal deposition. The method optionally comprises further steps in-between the named steps such as rinsing, drying or pretreatment steps which are known in the art.

Any substrate may be used in the method according to the invention. Typical substrates are conductive and semi-conductive substrates. Conductive substrates are metallic substrates including metallic seed layers as well (e.g. palladium deposited on a typical non-conductive substrate such as plastics to make the latter receptive for metal plating); semi-conductive substrates are exemplarily silicon and glass substrates. The substrate has at least one surface suitable to be treated with the inventive plating bath. The substrates are made in their entirety of the materials listed above or they only comprise one or more surfaces made of the materials listed above. It is also possible within the meaning of the present invention to treat more than one surface simultaneously or subsequently.

Preferably, the substrate is selected from the group consisting of printed circuit boards, IC substrates, circuit carriers, interconnect devices, semiconducting wafers and glass substrates. More preferred are substrates of the afore-mentioned group which have one or more recessed structures such as trenches, blind micro vias, through silicon vias and through glass vias. The metal or metal alloy, preferably copper or copper alloys, are then deposited into the recessed structures.

The deposition composition according to the invention is operated from 10 to 100°C for any time sufficient to deposit the desired deposit thickness. Suitable temperatures and durations can be determined by the person skilled in the art by routine experiments.

The plating bath can be either used in conventional vertical or horizontal plating equipment. Alternatively, fountain plating equipment may be used. The substrate or at least a portion of the surface may be contacted with the inventive plating bath by means of spraying, wiping, dipping, immersing or by other suitable means. Thereby, a metal or metal alloy deposit, preferably a copper or copper alloy deposit, is formed on at least a portion of the surface of the substrate.

It is preferential to agitate the deposition composition or the substrate during the plating process, *i.e.* the deposition of metal or metal alloy. Agitation may be accomplished for example by mechanical movement of the deposition composition like shaking, rotation, stirring; by mechanical movement of the substrate in the deposition composition such as rotation thereof or by continuously pumping of the liquids or by ultrasonic treatment, elevated temperatures or gas feeds (such as purging the deposition composition with air or an inert gas such as argon or nitrogen).

Optionally, the at least one substrate is subjected to one or more pre-treatment steps. Pre-treatment steps are known in the art and can be chosen by the person skilled in dependency of the substrate to be treated, the metal to be deposited and so forth. The pre-treatment steps can be for example cleaning steps, (micro-) etching steps and activation steps. Cleaning steps typically use aqueous solutions comprising one or more surfactants and are used to remove contaminants, e.g. from the at least one surface of the substrate which are detrimental to the metal plating deposition. (Micro-) Etching steps usually employ acidic solutions, optionally comprising one or more oxidant such as hydrogen peroxide, to increase the surface area of the at least one surface of the substrate. Activation steps usually require the deposition of a metal catalyst, most often palladium, on the at least one surface of the at least one substrate to render said at least one surface more receptive for metal deposition. Sometimes an activation step is preceded by a pre-dip step or succeeded by a post-dip step, both which are known in the art.

If optional step (iii) is included in the method according to the invention, the inventive deposition composition can be used for DC plating and (reverse) pulse plating. Both inert and soluble anodes can be utilized when depositing the metal or metal alloy from the deposition composition according to the present invention.

The preferred copper plating bath is preferably operated in the method according to the present invention in a temperature range of 15 °C to 50 °C, more preferably in a temperature range of 20 °C to 40 °C by applying an electrical current to the substrate and at least one anode. Preferably, a cathodic current density range of 0.05 A/dm² to 12 A/dm², more preferably 0.1 A/dm² to 7 A/dm² is applied.

In one embodiment of the present invention which is particularly suitable if the low concentration copper plating bath is used in the method according to the invention is as follows: first a low current density (e.g. 0.5 to 5 mA/cm², i.e. 0.05 to 0.5 A/dm²) is used for example to fill recessed structures followed by one or more high current density steps wherein each of said steps has a current density higher than that of the first low current density step which is useful for the overburden stage (see also the method disclosed in US 2014/209476 A1, in particular paragraphs 69-74 and Figure 7 thereof). E.g. the current density in the one or more high current density steps ranges preferably from 10 to 20 or 10 to 30 mA/cm² (1 to 2 A/dm² or 1 to 3 A/dm²). Optionally, the low current density step is preceded by a hot entry step which employs a high current density for a very short period of time, e.g. 15 to 20 mA/cm² for 0.05 to 1 s.

It is an advantage of the present invention that the copper plating bath according to the invention allows for uniformal filling of recessed structures and that the deposits are free of voids and dimples. Uniformal filling is to be understood in the context of the present invention that different recessed structures having different aspect ratios such as trenches which generally have an aspect ratio of < 1 and vias generally which have an aspect ratio of > 1 can be filled in one step resulting in similar layer distribution in these different recessed structures having different aspect ratios.

It is a further advantage of the present invention that the copper plating bath allows for optimal mounding results to be obtained. Mounding means that a planar copper deposit can be obtained that does neither show any dimples nor any overburdens.

Preferably, in case of the copper plating bath according to the invention, pure copper is deposited (disregarding any trace impurities commonly present in technical raw materials). Pure copper is particularly useful in the semiconductor industry due to its high conductivity. Pure copper shall be understood in the context of the present invention that a minimum copper content of 95 wt.-% based on the entire metal content of a deposit is formed, preferably 98 wt.-%, more preferably 99.0 wt.-%, most preferably 99.90 wt.-%. In a more preferred embodiment, the deposits formed consist of at least 95 wt.-% copper, preferably at least of 99 wt.-% copper, more preferably at least of 99.9 wt.-% copper.

The invention will now be illustrated by reference to the following non-limiting examples.

### Examples

Commercial products were used as described in the technical datasheet available on the date of filing of this specification unless stated otherwise hereinafter. Atomplate® accelerator and Atomplate® leveler are products available from Atotech Deutschland GmbH. Commercial products were used as described in the technical datasheet available on the date of filing of this specification unless stated otherwise hereinafter.

¹H-NMR spectra were recorded at 400 MHz with a spectrum offset of 4300 Hz, a sweep width of 9542 Hz at 25 °C (NMR spectroscope provided by Bruker Corp.). The solvent used was d⁶-DMSO unless stated otherwise.

The weight average molecular mass M_{w} of the ureylene additives was determined by gel permeation chromatography (GPC) using the following GPC apparatus and conditions: SECurity GPC System PSS, pump: Agilent 1260, column: PL Aquagel OH 30+40, 40 °C, Eluent A: 60 vol.-% 0.3 mol/L with formic acid in water + 40 vol.-% methanol, flow: 1 mL/min, detector RI 40 °C, time: 30 minutes, injection volume: 50 microliter, calibration: PVP=Poly-(2vinylpyridin), calibration: 800-256000) PPS standards, data system: WIN GPC V8.0.

A FEI Helios Nanolab 450S was used as Focused Ion Beam-Scanning Electron Microscopy (FIB-SEM). Focused Ion Beam milling was used to generate high quality cross sections and high resolution scanning electron microscopy was used for imaging (back scattering, magnification of e.g. 50,000). Deposit thicknesses were obtained using this method. Additionally or alternatively, a Laser Scanning Confocal Microscope from Olympus (Olympus Lext OLS4100) was used to measure the overburden conformity of plated samples.

Alternatively, the deposit thickness was measured at 10 positions of each substrate and was used to determine the layer thickness by XRF using the XRF instrument Fischerscope XDV-SDD (Helmut Fischer GmbH, Germany). By assuming a layered structure of the deposit, the layer thickness can be calculated from such XRF data.

As substrates for plating experiments the following wafers were used (provided by SUNY Polytechnic Institute):
- Substrate 1: TEOS backfill Empire II M1 wafer: 3 nm PVD TaN / 2 nm CVD Co/ 20nm PVD Cu. Depth: 150nm; pitch: 84nm (PVD = physical vapor deposition)
- Substrate 2: Empire II M2 wafer: 3 nm PVD TaN / 3 nm PVD Ta / 15nm PVD Cu. Depth: 200nm; pitch: 84nm
- Substrate 3: Empire M1 wafer: 3 nm PVD TaN / 3 nm PVD Ta / 30nm PVD Cu Alloy. Depth: 200nm; pitch: 200nm.

### Preparation of intermediate I

56.9 g Surfonamine B-60 was dissolved in 100 mL chloroform while stirring at 300 rpm (rounds per minute). This solution was cooled to 20 °C and maintained at the given temperature while 8.08 mL 1-chloro-2-isocyanatoethane in 50 mL chloroform was added. The reaction mixture was stirred for 20 hours at 20 °C. The solvent was then removed under reduced pressure until weight constancy was reached. The intermediate I was obtained as golden-orange oil (66.8 g, quantitative yield).
¹H NMR (500 MHz, Chloroform-d) δ 5.87 - 4.57 (m, 2H), 3.88 (s, 3H), 3.45 (dd, J = 89.8, 19.7 Hz, 39H), 3.33 (s, 3H), 3.30 (s, 1H), 1.12 (s, 33H).

### Preparation of intermediate II

18.95 g Surfonamine L-207 was dissolved in 60 mL chloroform while stirring at 300 rpm. This solution was cooled to 20 °C and maintained at the given temperature while 0.81 mL 1-chloro-2-isocyanatoethane was added. The reaction mixture was stirred for 20 hours at 20 °C. The solvent was then removed under reduced pressure followed until weight constancy was reached. The intermediate II was obtained as clear and colorless viscous oil (20.09 g, quantitative yield).
¹H NMR (500 MHz, Chloroform-d) δ 3.86 (s, 1H), 3.79 - 3.34 (m, 119H), 3.33 (s, 3H), 1.11 (s, 22H).

### Preparation of intermediate III

40.0 g Surfonamine L-100 was dissolved in 80 mL chloroform while stirring at 300 rpm. This solution was cooled to 20 °C and maintained at the given temperature while 4.2 g 1-chloro-2-isocyanatoethane was added. The reaction mixture was stirred for 20 hours at 20 °C. The solvent was then removed under reduced pressure until weight constancy was reached. The intermediate III was obtained as clear and waxy white solid (44.8 g, quantitative yield).
¹H NMR (500 MHz, Chloroform-d) δ 3.77 - 3.53 (m, 97H), 3.53 - 3.36 (m, 11H), 3.34 (s, 3H), 1.16 -0.96 (m, 9H).

### Further intermediates

Following the procedure given for intermediate I, further intermediates were prepared. A summary of all intermediates is given in the subsequent table 1.

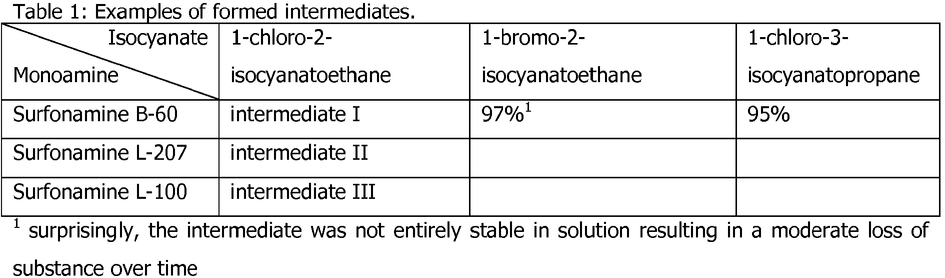

It can be seen that the yield decreased slightly when using 1-chloro-3-isocyanatopropane instead of 1-chloro-2-isocyanatoethane. Shorter alkanediyl chains in the isocyanate appear thus preferable in terms of yields.

### Preparation of multifunctional starting material I

6.82 mL *N¹*,*N¹*-dimethylpropane-1,3-diamine was dissolved in 40 mL chloroform and stirred at 300 rpm while cooling to 20 °C. Then, a solution of 4.36 mL 1,6-diisocyanatohexane in 40 mL chloroform was added slowly. The reaction mixture was stirred overnight at 20 °C whereby a precipitate was formed. Said precipitate was collected by suction filtration using additional chloroform to wash it. Finally, the precipitate was dried in-vacuo at 20 °C. The multifunctional starting material I was obtained as fluffy white powder (9.85 g, 99%).
¹H NMR (500 MHz, Deuterium Oxide) δ 3.09 (dt, J = 12.9, 6.8 Hz, 8H), 2.39 - 2.30 (m, 4H), 2.19 (s, 12H), 1.64 (p, J = 6.9 Hz, 4H), 1.51 - 1.41 (m, 4H), 1.31 (s, 4H).

### Preparation of urevlene additive I

A flask was charged with 5.0 g intermediate I and 0.226 g 2,2'-oxybis(*N*,*N*-dimethylethan-1-amine) prior to adding 40 mL acetonitrile. Under stirring at 300 rpm, the reaction mixture was kept at 80 °C for five days. The solvent was then removed under reduced pressure. The ureylene additive I was obtained as yellow oil (5.42 g, 97%).
¹H NMR (500 MHz, Deuterium Oxide) δ 3.87 - 3.39 (m, 67H), 3.38 (s, 7H), 3.34 (s, 3H), 3.12 (s, 10H), 2.86 (s, 1H), 2.63 (s, 2H), 1.74 (d, J = 79.3 Hz, 4H), 1.45 (s, 4H), 1.16 (s, 54H).

### Preparation of urevlene additive II

A flask was charged with 5.0 g intermediate II and 0.61 g *N¹*,*N¹*,*N⁶*,*N⁶*-tetramethylhexane-1,6-diamine prior to adding 40 mL acetonitrile. While stirring at 300 rpm, the reaction mixture was kept at 80 °C overnight. Then, the solvent was then removed under reduced pressure until weight constancy was reached. The ureylene additive II was obtained as yellow oil (5.19 g, quantitative yield).
¹H NMR (500 MHz, Chloroform-d) δ 4.17 (d, J = 20.3 Hz, 4H), 3.97 (d, J = 17.6 Hz, 4H), 3.85 (s, 3H), 3.79 - 3.36 (m, 309H), 3.35 (s, 9H), 2.85 (s, 3H), 1.12 (d, J = 6.3 Hz, 54H).

### Preparation of ureylene additive III

A flask was charged with 5.0 g intermediate I and 0.84 g multifunctional starting material I prior to adding 40 mL acetonitrile. While stirring at 300 rpm, the reaction mixture was kept at 80 °C overnight. Then, the solvent was removed under reduced pressure. The ureylene additive III was obtained as viscous and tacky orange oil (6.32 g, quantitative yield).
¹H NMR (500 MHz, Deuterium Oxide) δ 3.84 - 3.31 (m, 68H), 3.30 (s, 8H), 3.27 (s, 2H), 3.13 (s, 3H), 3.04 (s, 10H), 3.00 (s, 4H), 1.89 (s, 4H), 1.39 (s, 4H), 1.23 (s, 4H), 1.07 (s, 54H).

### Preparation of ureylene additive IV

A flask was charged with 4.2 g intermediate III and 0.71 g multifunctional starting material I prior to adding 40 mL acetonitrile. While stirring at 300 rpm, the reaction mixture was kept at 80 °C overnight. Then, the solvent was then removed under reduced pressure. The ureylene additive IV was obtained as pale yellow waxy solid (4.80 g, 98%).
¹H NMR (500 MHz, Deuterium Oxide) δ 3.71 (s, 212H), 3.39 (s, 10H), 3.21 (s, 6H), 3.13 (s, 10H), 3.09 (s, 6H), 2.86 (s, 6H), 1.98 (s, 4H), 1.47 (s, 5H), 1.32 (s, 6H), 1.15 (s, 18H).

### Preparation of further ureylene additives

Following the procedure given for ureylene additive I, further ureylene additives were prepared from the intermediates and multifunctional starting materials (MSM). A summary of all ureylene additives is given in the subsequent table 2. In the column right of the individual chemical structures, it is outlined to which type of multifunctional starting materials (MSM) the chemical structure belongs.

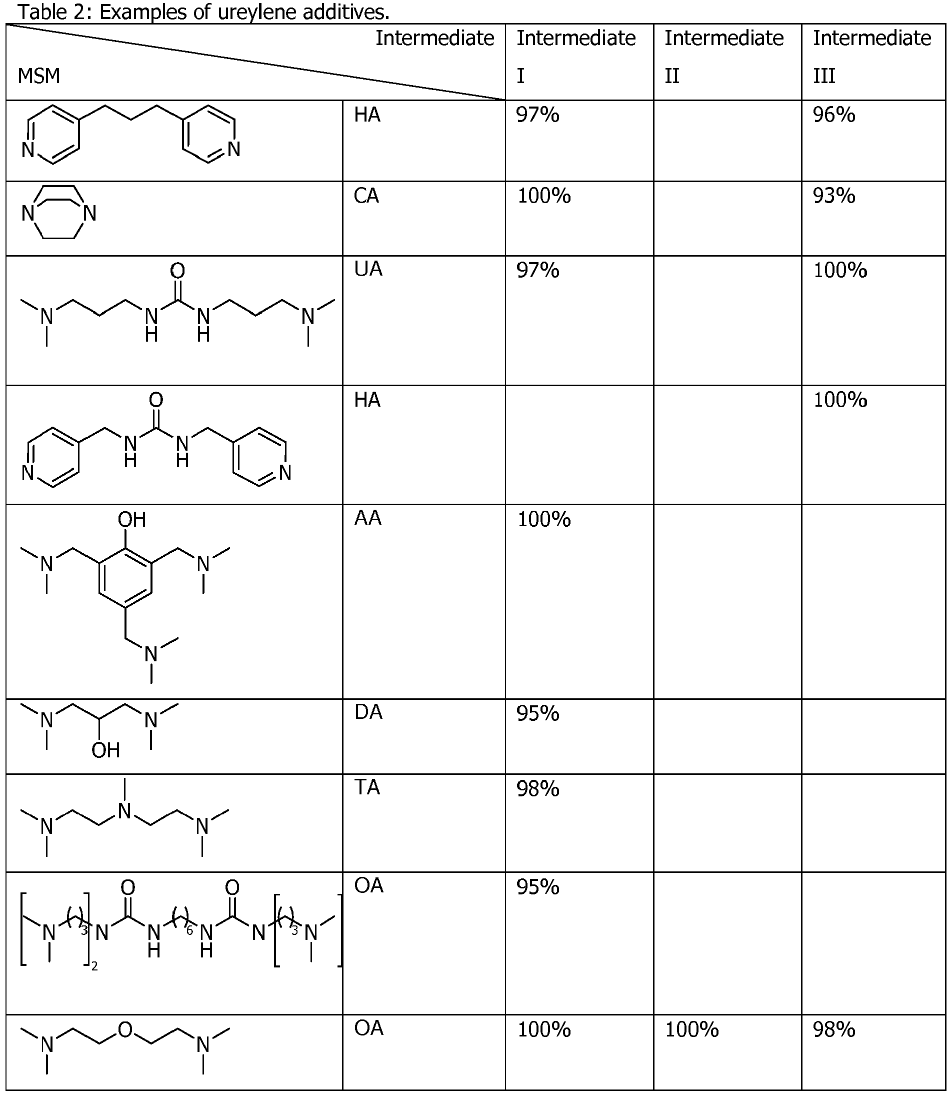

Some of the ureylene additives were chosen for plating experiments which are described hereinafter. However, it is believed by the inventors that also the other ureylene additives perform similarly in a metal or metal alloy deposition composition.

### Plating experiment 1

Solutions each comprising 5 g/L Cu²⁺ ions (added as copper sulfate), 10 g/L sulfuric acid, 50 mg/L chloride ions (added as hydrochloric acid), Atomplate® leveler, Atomplate® accelerator (all products of Atotech Deutschland GmbH) and ureylene additive I in concentrations as given below were used.

Substrates consisting of coupons of approximately 20 mm x 25 mm cleaved from a 300 mm 28 nm node test pattern wafer provided by SUNY Polytechnic Institute. All features were 150-200 nm deep. Features used for these experiments had a line to space ratio of 1:1 and had a pitch of 84 nm. The barrier/liner/seed stack consisted of 3 nm PVD TaN / 3 nm PVD Ta/ 15nm PVD Cu for Empire II M2 and 3 nm PVD TaN / 2 nm CVD Co/ 20nm PVD Cu for TEOS backfill Empire II M1. The coupons were plated in a beaker-scale coupon plating apparatus where the temperature was 25 °C, and the anode was a soluble copper anode separated by a nafion membrane from the cathode chamber. The waveform consisted of an entry step followed by four distinct plating steps. The entry step used a potential controlled hot entry to match a nominal 16 mA/cm² at 90 rpm for 0.1 s. The first plating step was 6.5 mA/cm² at 60 rpm for 5.5 s. The second plating step was at 10 mA/cm² at 60 rpm for 20 s. The third plating step was at 10 mA/cm² at 225 rpm for 4.4 s. The fourth plating step was at 22 mA/cm² at 225 RPM for 46 s. The final thickness of the deposit was approximately 470 nm. Afterwards, each substrate was also rinsed with DI water and dried by nitrogen gas flow such that dried substrates were obtained. The filling performance was evaluated with FIB-SEM (FEI Helios Nanolab 450S). A slope cut was carried to check the voids in the plated trench.

**Table 3: Plating results overview.**

| Plating experiment | Concentration [mg/L] | | | Filling | Voids |
|---|---|---|---|---|---|
| | Atomplate® accelerator | Atomplate® leveler | ureylene additive I | | |
| 1.1 | 24 | 3.5 | 100 | full | none |
| 1.2 | 48 | 3.5 | 100 | full | none |
| 1.3 | 48 | 3.5 | 200 | full | none |
| 1.4 | 48 | 3.5 | 300 | full | none |
| 1.5 | 72 | 3.5 | 300 | full | none |

### Plating experiment 2

Plating experiment 1 was repeated while using ureylene additive II instead of ureylene additive I.

**Table 4: Plating results overview.**

| Plating experiment | Concentration [mg/L] | | | Filling | Voids |
|---|---|---|---|---|---|
| | Atomplate® accelerator | Atomplate® leveler | ureylene additive II | | |
| 2.1 | 24 | 3.5 | 100 | full | none |
| 2.2 | 48 | 3.5 | 100 | full | none |
| 2.3 | 48 | 3.5 | 200 | full | none |
| 2.4 | 48 | 3.5 | 300 | full | none |
| 2.5 | 72 | 3.5 | 300 | full | none |

### Plating experiment 3

Plating experiment 1 was repeated while using ureylene additive III instead of ureylene additive I.

**Table 5: Plating results overview.**

| Plating experiment | Concentration [mg/L] | | | Filling | Voids |
|---|---|---|---|---|---|
| | Atomplate® accelerator | Atomplate® leveler | ureylene additive III | | |
| 3.1 | 24 | 3.5 | 100 | full | none |
| 3.2 | 48 | 3.5 | 100 | full | none |
| 3.3 | 48 | 3.5 | 200 | full | none |
| 3.4 | 48 | 3.5 | 300 | full | none |
| 3.5 | 72 | 3.5 | 300 | full | none |

### Plating experiment 4

Plating experiment 1 was repeated while using ureylene additive IV instead of ureylene additive I.

**Table 6: Plating results overview.**

| Plating experiment | Concentration [mg/L] | | | Filling | Voids |
|---|---|---|---|---|---|
| | Atomplate® accelerator | Atomplate® leveler | ureylene additive IV | | |
| 4.1 | 24 | 3.5 | 100 | full | none |
| 4.2 | 48 | 3.5 | 100 | full | none |
| 4.3 | 48 | 3.5 | 200 | full | none |
| 4.4 | 48 | 3.5 | 300 | full | none |
| 4.5 | 72 | 3.5 | 300 | full | none |

All of the inventive plating experiments 1 to 4 showed full filling of the recessed structures with copper and the copper deposits were free of voids as required in the electronics and semiconductor industry.

### Comparative Plating experiment C1

Plating experiment 1 was repeated but the ureylene additive I was substituted by a standard polyethylene imine suppressor.

The filling was incomplete and many voids were found in the filling of the recessed structure. Such a copper deposit is unsuitable for industrial applications.

### Plating experiment 5: Partial fill

Solutions each comprising 5 g/L Cu²⁺ ions (added as copper sulfate), 10 g/L sulfuric acid, 50 mg/L chloride ions (added as hydrochloric acid), 3.5 mg/L Atomplate® leveler, 48 mg/L Atomplate® accelerator (all products of Atotech Deutschland GmbH) and the respective ureylene additives in concentrations as given below were used.

The partial fill was carried on Empire M1 wafers provided by SUNY Polytechnic Institute. Features used for these experiments had a line to space ratio of 1:1 and opening dimensions of approximately 100 nm and a depth of approximately 200 nm. The barrier/liner/seed stack consisted of 3 nm PVD TaN / 3 nm PVD Ta/ 30nm PVD Cu Alloy. The waveform consisted of an entry step followed by a partial fill plating step. The entry step used a potential controlled hot entry to match a nominal 16 mA/cm² at 135 rpm for 0.1 s. The partial fill plating step was carried at 6.5 mA/cm² under 60 rpm for 4 s. A transverse cut was carried to measure the filling height in the trench (given as normalized fill height in Table 7).

**Table 7: Partial fill plating results.**

| Plating experiment | Additive | c [mg/L] | Normalized fill height |
|---|---|---|---|
| 5.1 | Ureylene additive I | 100 | 0.63 |
| 5.2 | Ureylene additive I | 200 | 0.44 |
| 5.3 | Ureylene additive II | 100 | 0.56 |
| 5.4 | Ureylene additive II | 200 | 0.43 |
| 5.5 | Ureylene additive IV | 100 | 0.57 |
| 5.6 | Ureylene additive IV | 200 | 0.43 |

All the copper deposits were free of voids.

### Comparative plating experiment C2: Partial fill

Plating experiment 5 was repeated with 100 mg/L polyethylene imine as standard suppressor instead of the ureylene additives in plating experiment 5. The normalized fill height was 0.37 for 100ppm and 0.35 for 200ppm.

The inventive examples 5.1 to 5.6 using copper plating baths containing ureylene additives allowed for improved normalized fill heights under otherwise identical experimental conditions compared to the comparative plating experiment C2. They, therefore, enhance the plating rate of the copper deposition which is highly desired in the electronics and semiconductor industry. Further, the inventive copper plating baths allowed for copper deposits without voids to be formed.

Other embodiments of the present invention will be apparent to those skilled in the art from a consideration of this specification or practice of the invention disclosed herein. It is intended that the specification and examples be considered as exemplary only, with the true scope of the invention being defined by the following claims only.

### Industrial Applicability

The present invention can preferably be used in the manufacturing processes of the electronics and semiconductor industry.

## Claims

1. An ureylene additive comprising at least one structural unit represented by formula (I) 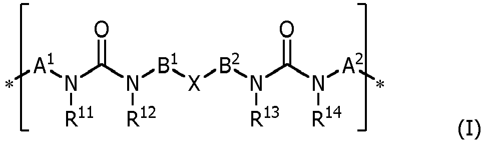 wherein
A¹ and A² are independently selected from the group consisting of oligo(oxyalkanediyl) group and poly(oxyalkanediyl) group;
B¹ and B² are independently alkanediyl groups;
R¹¹, R¹², R¹³ and R¹⁴ are independently selected from the group consisting of hydrogen, alkyl group and aryl group; and
X is selected from the group consisting of
- structural units represented by formula (II) 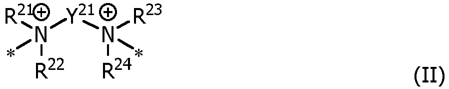 wherein
R²¹, R²², R²³ and R²⁴ are independently selected from the group consisting of alkyl group and aryl group; and
Y²¹ is selected from the group consisting of alkanediyl group, alkanediyloxyalkanediyl group, alkanediyloligo(oxyalkanediyl) group and alkanediylpoly(oxyalkanediyl) group;
- structural units represented by formula (III) 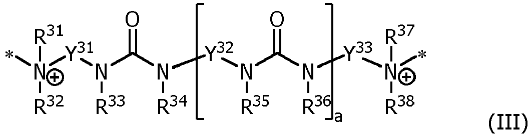 wherein
R³¹, R³², R³⁷ and R³⁸ are independently selected from the group consisting of alkyl group and aryl group;
R³³, R³⁴, each R³⁵ and each R³⁶ are independently selected from the group consisting of hydrogen, alkyl group and aryl group;
Y³¹ and Y³³ are independently alkanediyl groups;
each Y³² is independently selected from the group consisting of alkanediyl group, alkanediyloxyalkanediyl group, alkanediyloligo(oxyalkanediyl) group and alkanediylpoly(oxyalkanediyl) group; and
a is an integer selected from the group consisting of 0, 1, 2 and 3;
- structural units represented by formula (IV) 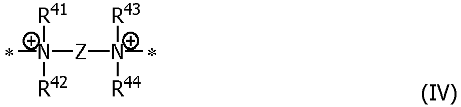 wherein
R⁴¹, R⁴², R⁴³ and R⁴⁴ are independently selected from the group consisting of alkyl group and aryl group;
Z is represented by one of the following formulae (Z1) to (Z3) 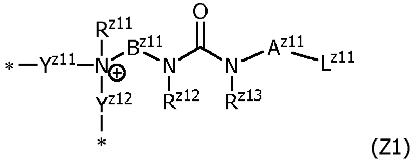 wherein
Y^{z11} and Y^{z12} are independently alkanediyl groups;
A^{z11} is independently selected from the group consisting of oligo(oxyalkanediyl) group and poly(oxyalkanediyl) group;
B^{z11} is independently selected to be an alkanediyl group;
R^{z11} is selected from the group consisting of alkyl group and aryl group;
R^{z12} and R^{z13} are independently selected from the group consisting of hydrogen, alkyl group and aryl group;
L^{z11} is selected from the group consisting of hydrogen, alkyl group and aryl group; 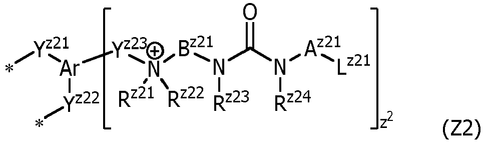
wherein
Ar is an aromatic group;
Y^{z21}, Y^{z22} and each Y^{z23} independently are alkanediyl groups;
each A^{z21} is independently selected from the group consisting of oligo(oxyalkanediyl) group and poly(oxyalkanediyl) group;
each B^{z21} is independently selected to be an alkanediyl group;
each R^{z21} and each ^{Rz22} are independently selected from the group consisting of alkyl group and aryl group;
each R^{z23} and each R^{z24} are independently selected from the group consisting of hydrogen, alkyl group and aryl group;
each L^{z21} is independently selected from the group consisting of hydrogen, alkyl group and aryl group;
z² is an integer selected from 1 and 2;
and 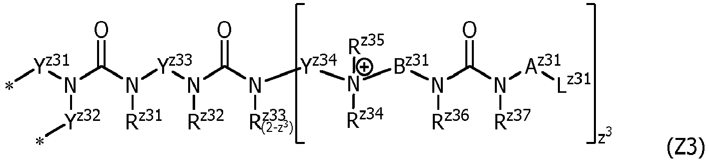
wherein
Y^{z31}, Y^{z32}, Y^{z33} and each Y^{z34} are independently alkanediyl groups;
each A^{z31} is independently selected from the group consisting of oligo(oxyalkanediyl) group and poly(oxyalkanediyl) group;
each B^{z31} is independently selected to be an alkanediyl group;
R^{z31}, R^{z32}, R^{z33}, each R^{z36} and each R^{z37} are independently selected from the group consisting of hydrogen, alkyl group and aryl group;
each R^{z34} and each R^{z35} are independently selected from the group consisting of alkyl group and aryl group;
each L^{z31} is independently selected from the group consisting of hydrogen, alkyl group and aryl group;
z³ is an integer selected from 1 and 2;
- structural units represented by formula (V) 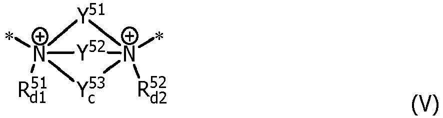 wherein
each R⁵¹ and each R⁵² are independently selected from the group consisting of alkyl group and aryl group;
each of Y⁵¹, Y⁵² and Y⁵³ is independently an alkanediyl group;
c is selected from 0 and 1;
d1 is an integer expressed by the mathematical equation d1=(1-c);
d2 is an integer expressed by the mathematical equation d2=(1-c); and
and
- structural units represented by formula (VI) 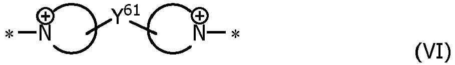 wherein
Y⁶¹ is selected from the group consisting of alkanediyl group and alkanediyl-urediyl-alkanediyl group.

2. The ureylene additive according to claim 1 **characterized in that** A¹, A², A^{z11}, A^{z21} and A^{z31} are independently represented by the following formula (A-1) 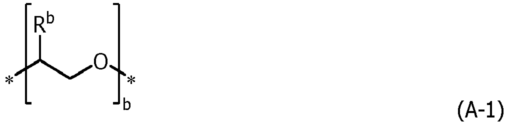 wherein each R^{b} is selected from the group consisting of hydrogen, alkyl group and aryl group; preferably from hydrogen and C1-C4-alkyl group; more preferably from hydrogen and methyl group;
b is an integer ranging from 3 to 100, preferably 5 to 75, more preferably 7 to 50.

3. The ureylene additive according to any one of the preceding claims **characterized in that** B¹, B², B^{z11}, B^{z21} and B^{z31} are independently a C1-C8-alkanediyl group, preferably a C2-C6-alkanediyl group, more preferably a C2-C4-alkanediyl group, they are even more preferably selected from the group consisting of ethane-1,2-diyl and propane-1,3-diyl; they are most preferably ethane-1,2-diyl.

4. The ureylene additive according to any one of the preceding claims **characterized in that** it comprises a capping group L¹¹ linked to A¹ and/or a capping group L¹² linked to A² wherein the capping groups L¹¹ and/or L¹² are independently selected from the group consisting of hydrogen, alkyl group and aryl group; L¹¹ and/or L¹² are preferably independently alkyl groups, more preferably C1-C4-alkyl groups, even more preferably C1-C2-alkyl groups, still even more preferably methyl groups.

5. The ureylene additive according to any one of the preceding claims **characterized in that** R¹¹, R¹², R¹³, R¹⁴, R³³, R³⁴, R³⁵, R³⁶, R^{z12}, R^{z13}, R^{z23}, R^{z24}, R^{z31}, R^{z32}, R^{z33}, R^{z36} and R^{z37} are independently selected from the group consisting of hydrogen and C1-C4-alkyl group; more preferably they are hydrogen.

6. The ureylene additive according to any one of the preceding claims **characterized in that** R²¹, R²², R²³, R²⁴, R³¹, R³², R³⁷, R³⁸, R⁴¹, R⁴², R⁴³, R⁴⁴, R⁵¹ R⁵², R^{z11}, R^{z21}, R^{z22}, R^{z34} and R^{z35} are independently of each other selected are independently C1-C4-alkyl groups; more preferably C1-C2-alkyl groups; most preferably they are methyl groups.

7. The ureylene additive according to any one of the preceding claims **characterized in that** X is selected from structural units represented by formula (II) and structural units represented by formula (III), preferably X is a structural unit represented by formula (III).

8. A method for preparing an ureylene additive by reacting at least one isocyanate represented by formula (ISO) 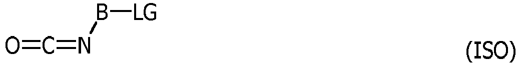 wherein
B is an alkanediyl group; and
LG is a leaving group suitable to be replaced by nucleophilic substitution;
with at least one monoamine comprising a structural unit represented by formula (MA) 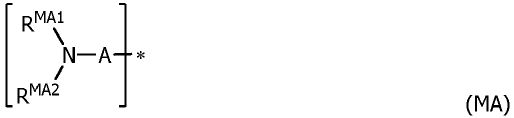 wherein
A is selected from the group consisting of oligo(oxyalkanediyl) group and poly(oxyalkanediyl) group; and
each R^{MA1} and R^{MA2} is selected from the group consisting of hydrogen, alkyl group and aryl group;
and thereby forming at least one intermediate;
and said at least one intermediate is reacted with at least one multifunctional starting material selected from the group consisting of diamines, ureaamines, triamines, aromatic amines, diureaoligoamines, cyclic amines and heterocyclic amines
wherein
the diamines are represented by formula (DA) 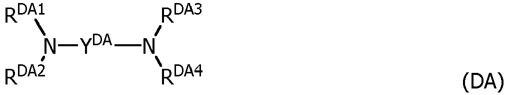
wherein
R^{DA1}, R^{DA2}, R^{DA3} and R^{DA4} are independently selected from the group consisting of alkyl group and aryl group; and
Y^{DA} is selected from the group consisting of alkanediyl group, alkanediyloxyalkanediyl group, alkanediyloligo(oxyalkanediyl) group, alkanediylpoly(oxyalkanediyl) group;
the ureaamines are represented by formula (UA) 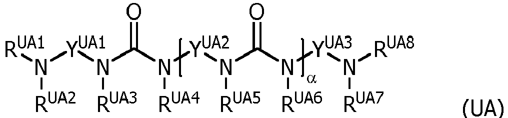 wherein
R^{UA1}, R^{UA2}, R^{UA7} and R^{UA8} are independently selected from the group consisting of alkyl group and aryl group; and
R^{UA3}, R^{UA4}, each R^{UA5} and each R^{UA6} are independently selected from the group consisting of hydrogen, alkyl group and aryl group;
Y^{UA1} and Y^{UA3} are independently alkanediyl groups;
each Y^{UA2} is independently selected from the group consisting of alkanediyl group, alkanediyloxyalkanediyl group, alkanediyloligo(oxyalkanediyl) group and alkanediylpoly(oxyalkanediyl) group; and
α is an integer selected from the group consisting of 0, 1, 2 and 3;
the triamines are represented by formula (TA) 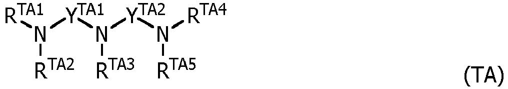 wherein
Y^{TA1} and Y^{TA2} independently are alkanediyl groups;
R^{TA1}, R^{TA2}, R^{TA3}, R^{TA4} and R^{TA5} are independently selected from the group consisting of alkyl group and aryl group;
the aromatic amines are represented by formula (AA) 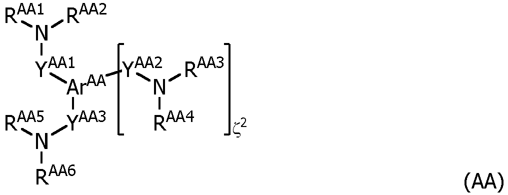 wherein
Ar^{AA} is an aromatic group;
Y^{AA1}, each Y^{AA2} and Y^{AA3} independently are alkanediyl groups;
R^{AA1}, R^{AA2}, R^{AA3}, R^{AA4}, R^{AA5}, R^{AA5} and R^{AA6} are independently selected from the group consisting of alkyl group and aryl group;
ζ² is an integer selected from 1 and 2;
the diureaoligoamines are represented by formula (OA) 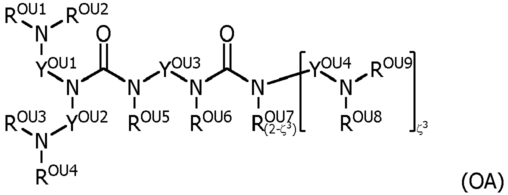 wherein
Y^{OU1}, Y^{OU2}, Y^{OU3} and each Y^{OU4} are independently alkanediyl groups;
R^{OU5}, R^{OU6} and each R^{OU7} are independently selected from the group consisting of hydrogen, alkyl group and aryl group;
R^{OU1}, R^{OU2}, R^{OU3}, R^{OU4}, each R^{OU8}, and each R^{OU9} are independently selected from the group consisting of alkyl group and aryl group;
ζ³ is an integer selected from 1 and 2; and
the cyclic amines are represented by formula (CA) 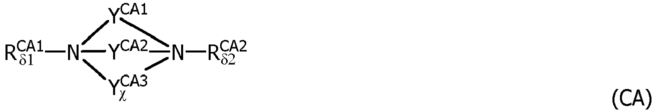 wherein
R^{CA1} and R^{CA2} is independently selected from the group consisting of alkyl group and aryl group;
Y^{CA1}, Y^{CA2} and Y^{CA3} are independently selected from the group consisting of alkanediyl; χ is an integer selected from 0 and 1; and
δ1 is an integer expressed by the mathematical equation δ1=(1-χ); δ2 is an integer expressed by the mathematical equation δ2=(1-χ);
the heterocyclic amines are represented by formula (HA) 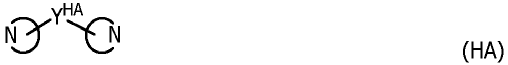 wherein
Y^{HA} is selected from the group consisting of alkanediyl group and alkanediyl-urediyl-alkanediyl group.

9. An intermediate useful for being reacted with a multifunctional starting material as defined in claim 8 to form a ureylene additive according to any one of claims 1 to 7, the intermediate comprising a structural unit represented by formula (INT) 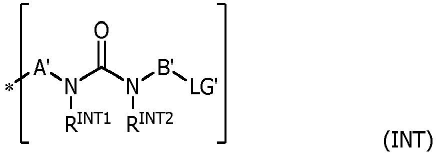 wherein
B' is an alkanediyl group;
LG' is a leaving group suitable to be replaced by nucleophilic substitution;
R^{INTI'} and R^{INT2'} are independently selected from the group consisting of hydrogen, alkyl group and aryl group;
A' is selected from the group consisting of oligo(oxyalkanediyl) group and poly(oxyalkanediyl) group.

10. Use of an ureylene additive according to any one of claim 1 to 7 in a metal or metal alloy deposition composition, preferably in a copper or copper alloy deposition composition, more preferably in an electrolytic copper or copper alloy deposition composition, in particular as leveler and/or suppressor therein.

11. A metal or metal alloy deposition composition comprising at least one type of reducible metal ions, preferably copper ions, and at least one ureylene additive according to any one of claims 1 to 7.

12. The metal or metal alloy deposition composition of claim 11 wherein the at least one type of reducible metal ions are copper ions.

13. Use of the metal or metal alloy composition according to any one of claims 11 or 12 to form a metal or metal alloy layer, preferably a copper or copper alloy layer, on a surface of a substrate.

14. A method for depositing a metal or metal alloy layer, preferably a copper or copper alloy layer, onto at least one surface of a substrate comprising the following method steps
(i) providing the substrate having said at least one surface
(ii) contacting said at least one surface of the substrate with the composition according to any one of claims 11 or 12;
(iii) optionally, applying an electrical current between the substrate and at least one anode; and thereby forming the metal or metal alloy layer, preferably the copper or copper alloy layer, on said at least one surface of the substrate.

## Patentansprüche

1. Ureylenadditiv, umfassend mindestens eine Struktureinheit, die durch Formel (I) wiedergegeben wird, 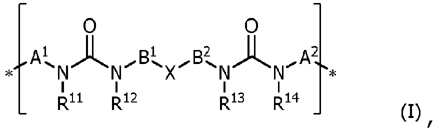 wobei
A¹ und A² unabhängig aus der Gruppe bestehend aus einer Oligo(oxyalkandiyl)gruppe und einer Poly-(oxyalkandiyl)gruppe ausgewählt sind;
B¹ und B² unabhängig für Alkandiylgruppen stehen; R¹¹, R¹², R¹³ und R¹⁴ unabhängig aus der Gruppe bestehend aus Wasserstoff, einer Alkylgruppe und einer Arylgruppe ausgewählt sind und
X aus der Gruppe bestehend aus
- Struktureinheiten, die durch Formel (II) wiedergegeben werden, 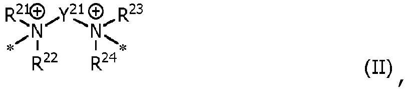 wobei
R²¹, R²², R²³ und R²⁴ unabhängig aus der Gruppe bestehend aus einer Alkylgruppe und einer Arylgruppe ausgewählt sind und
Y²¹ aus der Gruppe bestehend aus einer Alkandiylgruppe, einer Alkandiyloxyalkandiylgruppe, einer Alkandiyloligo(oxyalkandiyl)gruppe und einer Alkandiylpoly(oxyalkandiyl)gruppe ausgewählt ist;
- Struktureinheiten, die durch Formel (III) wiedergegeben werden, 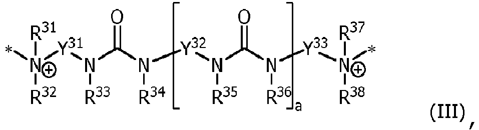 wobei
R³¹, R³², R³⁷ und R³⁸ unabhängig aus der Gruppe bestehend aus einer Alkylgruppe und einer Arylgruppe ausgewählt sind und
R³³, R³⁴, jedes R³⁵ und jedes R³⁶ unabhängig aus der Gruppe bestehend aus Wasserstoff, einer Alkylgruppe und einer Arylgruppe ausgewählt sind;
Y³¹ und Y³³ unabhängig für Alkandiylgruppen stehen;
jedes Y³² unabhängig aus der Gruppe bestehend aus einer Alkandiylgruppe, einer Alkandiyloxyalkandiylgruppe, einer Alkandiyloligo(oxyalkandiyl)gruppe und einer Alkandiylpoly(oxyalkandiyl)gruppe ausgewählt ist und
a für eine ganze Zahl aus der Gruppe bestehend aus 0, 1, 2 und 3 steht;
- Struktureinheiten, die durch Formel (IV) wiedergegeben werden, 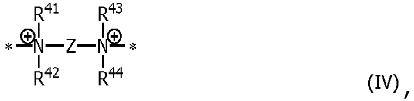 wobei
R⁴¹, R⁴², R⁴³ und R⁴⁴ unabhängig aus der Gruppe bestehend aus einer Alkylgruppe und einer Arylgruppe ausgewählt sind;
Z durch eine der folgenden Formeln (Z1) bis (Z3) wiedergegeben wird, 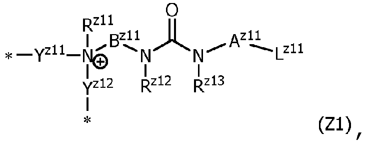 wobei
Y^{z11} und Y^{z12} unabhängig für Alkandiylgruppen stehen;
A^{z11} unabhängig aus der Gruppe bestehend aus einer Oligo(oxyalkandiyl)gruppe und einer Poly(oxyalkandiyl)gruppe ausgewählt ist;
B^{z11} so unabhängig ausgewählt ist, dass es für eine Alkandiylgruppe steht;
R^{z11} aus der Gruppe bestehend aus einer Alkylgruppe und einer Arylengruppe ausgewählt ist;
R^{z12} und R^{z13} unabhängig aus der Gruppe bestehend aus Wasserstoff, einer Alkylgruppe und einer Arylgruppe ausgewählt sind;
L^{z11} aus der Gruppe bestehend aus Wasserstoff, einer Alkylgruppe und einer Arylgruppe ausgewählt ist; 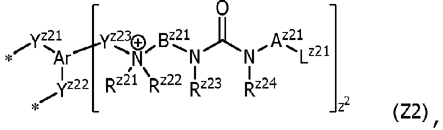 wobei
Ar für eine aromatische Gruppe steht;
Y^{z21}, Y^{z22} und jedes Y^{z23} unabhängig für Alkandiylgruppen stehen;
jedes A^{z21} unabhängig aus der Gruppe bestehend aus einer Oligo(oxyalkandiyl)gruppe und einer Poly(oxyalkandiyl)gruppe ausgewählt ist; jedes B^{z21} so ausgewählt ist, dass es für eine Alkandiylgruppe steht;
jedes R^{z21} und jedes R^{z22} unabhängig aus der Gruppe bestehend aus einer Alkylgruppe und einer Arylgruppe ausgewählt sind;
jedes R^{z23} und jedes R^{z24} unabhängig aus der Gruppe bestehend aus Wasserstoff, einer Alkylgruppe und einer Arylgruppe ausgewählt sind;
z² für eine ganze Zahl steht, die aus 1 und 2 ausgewählt ist;
und 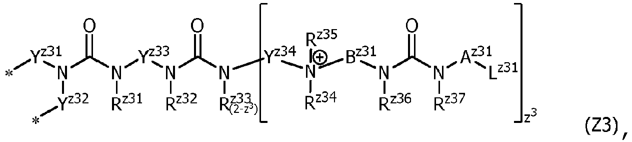 wobei
Y^{z31}, Y^{z32}, Y^{z33} und jedes Y^{z34} unabhängig für Alkandiylgruppen stehen;
jedes A^{z31} unabhängig aus der Gruppe bestehend aus einer Oligo(oxyalkandiyl)gruppe und einer Poly(oxyalkandiyl)gruppe ausgewählt ist; jedes B^{z31} so unabhängig ausgewählt ist, dass es für eine Alkandiylgruppe steht;
R^{z31}, R^{z32}, R^{z33}, jedes R^{z36} und jedes R^{z37} unabhängig aus der Gruppe bestehend aus Wasserstoff, einer Alkylgruppe und einer Arylgruppe ausgewählt sind;
jedes R^{z34} und jedes R^{z35} unabhängig aus der Gruppe bestehend aus einer Alkylgruppe und einer Arylgruppe ausgewählt sind;
jedes L^{z31} unabhängig aus der Gruppe bestehend aus Wasserstoff, einer Alkylgruppe und einer Arylgruppe ausgewählt ist;
z³ für eine ganze Zahl steht, die aus 1 und 2 ausgewählt ist;
- Struktureinheiten, die durch Formel (V) wiedergegeben werden, 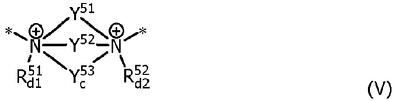 wobei
jedes R⁵¹ und jedes R⁵² unabhängig aus der Gruppe bestehend aus einer Alkylgruppe und einer Arylgruppe ausgewählt sind;
jedes von Y⁵¹, Y⁵² und Y⁵³ unabhängig für eine Alkandiylgruppe steht;
c aus 0 und 1 ausgewählt ist;
d1 für eine ganze Zahl steht, die durch die mathematische Gleichung d1 = (1-c) ausgedrückt wird;
d2 für eine ganze Zahl steht, die durch die mathematische Gleichung d2 = (1-c) ausgedrückt wird; und
und
- Struktureinheiten, die durch Formel (VI) wiedergegeben werden, 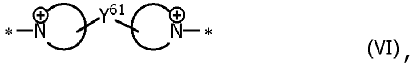 wobei
Y⁶¹ aus der Gruppe bestehend aus einer Alkandiylgruppe und einer Alkandiyl-urediyl-alkandiylgruppe ausgewählt ist;
ausgewählt ist.

2. Ureylenadditiv nach Anspruch 1, **dadurch gekennzeichnet, dass** A¹, A², A^{z11}, A^{z21} und A^{z31} unabhängig durch die folgende Formel (A-1) wiedergegeben werden, 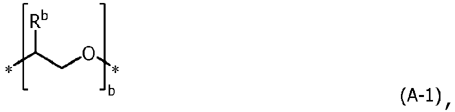 wobei jedes R^{b} aus der Gruppe bestehend aus Wasserstoff, einer Alkylgruppe und einer Arylgruppe, vorzugsweise aus Wasserstoff und einer C1-C4-Alkylgruppe, weiter bevorzugt aus Wasserstoff und einer Methylgruppe, ausgewählt ist;
b für eine ganze Zahl im Bereich von 3 bis 100, vorzugsweise 5 bis 75, weiter bevorzugt 7 bis 50, steht.

3. Ureylenadditiv nach einem der vorhergehenden Ansprüche, **dadurch gekennzeichnet, dass** B¹, B², B^{z11}, B^{z21} und B^{z31} unabhängig für eine C1-C8-Alkandiylgruppe, vorzugsweise eine C2-C6-Alkandiylgruppe, weiter bevorzugt eine C2-C4-Alkandiylgruppe, stehen und noch weiter bevorzugt aus der Gruppe bestehend aus Ethan-1,2-diyl und Propan-1,3-diyl ausgewählt sind und ganz besonders bevorzugt für Ethan-1,2-diyl stehen.

4. Ureylenadditiv nach einem der vorhergehenden Ansprüche, **dadurch gekennzeichnet, dass** es eine mit A¹ verknüpfte Verkappungsgruppe L¹¹ und/oder eine mit A² verknüpfte Verkappungsgruppe L¹² umfasst, wobei die Verkappungsgruppen L¹¹ und/oder L¹² unabhängig aus der Gruppe bestehend aus Wasserstoff, einer Alkylgruppe und einer Arylgruppe ausgewählt sind; L¹¹ und/oder L¹² vorzugsweise unabhängig für Alkylgruppen, weiter bevorzugt C1-C4-Alkylgruppen, noch weiter bevorzugt C1-C2-Alkylgruppen, noch weiter bevorzugt Methylgruppen, stehen.

5. Ureylenadditiv nach einem der vorhergehenden Ansprüche, **dadurch gekennzeichnet, dass** R¹¹, R¹², R¹³, R¹⁴, R³³, R³⁴, R³⁵, R³⁶, R^{z12}, R^{z13}, R^{z23}, R^{z24}, R^{z31}, R^{z32}, R^{z33}, R^{z36} und R^{z37} unabhängig aus der Gruppe bestehend aus Wasserstoff und einer C1-C4-Alkylgruppe ausgewählt sind und weiter bevorzugt für Wasserstoff stehen.

6. Ureylenadditiv nach einem der vorhergehenden Ansprüche, **dadurch gekennzeichnet, dass** R²¹, R²², R²³, R²⁴, R³¹, R³², R³⁷, R³⁸, R⁴¹, R⁴², R⁴³, R⁴⁴, R⁵¹, R⁵², R^{z11}, R^{z21}, R^{z22}, R^{z34} und R^{z35} unabhängig voneinander ausgewählt sind, unabhängig für C1-C4-Alkylgruppen, weiter bevorzugt C1-C2-Alkylgruppen, ganz besonders bevorzugt Methylgruppen, stehen.

7. Ureylenadditiv nach einem der vorhergehenden Ansprüche, **dadurch gekennzeichnet, dass** X aus Struktureinheiten, die durch Formel (II) wiedergegeben werden, und Struktureinheiten, die durch Formel (III) wiedergegeben werden, ausgewählt ist und vorzugsweise X für eine Struktureinheit, die durch Formel (III) wiedergegeben wird, steht.

8. Verfahren zur Herstellung eines Ureylenadditivs durch Umsetzen von mindestens einem Isocyanat, das durch Formel (ISO) wiedergegeben wird, 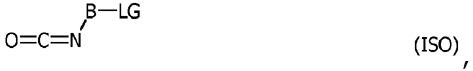 wobei
B für eine Alkandiylgruppe steht und
LG für eine durch nukleophile Substitution ersetzbare Abgangsgruppe steht;
mit
mindestens einem Monoamin mit einer Struktureinheit, die durch Formel (MA) wiedergegeben wird, 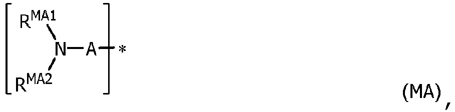 wobei
A aus der Gruppe bestehend aus einer Oligo(oxy-alkandiyl)gruppe und einer Poly(oxyalkandiyl)-gruppe ausgewählt ist und
jedes R^{MA1} und R^{MA2} aus der Gruppe bestehend aus Wasserstoff, einer Alkylgruppe und einer Arylgruppe ausgewählt ist;
oder durch mindestens ein Zwischenprodukt gebildet wird;
und das mindestens eine Zwischenprodukt mit mindestens einem multifunktionellen Ausgangsstoff aus der Gruppe bestehend aus Diaminen, Harnstoffaminen, Triaminen, aromatischen Aminen, Diharnstoffoligoaminen, cyclischen Aminen und heterocyclischen Aminen umgesetzt wird,
wobei
die Diamine durch Formel (DA) wiedergegeben werden, 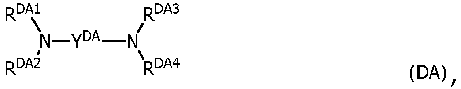 wobei
R^{DA1}, R^{DA2}, R^{DA3} und R^{DA4} unabhängig aus der Gruppe bestehend aus einer Alkylgruppe und einer Arylgruppe ausgewählt sind und
Y^{DA} aus der Gruppe bestehend aus einer Alkandiylgruppe, einer Alkandiyloxyalkandiylgruppe, einer Alkandiyloligo(oxyalkandiyl)gruppe und einer Alkandiylpoly(oxyalkandiyl)gruppe ausgewählt ist; die Harnstoffamine durch Formel (UA) wiedergegeben werden, 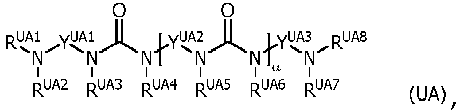 wobei
R^{UA1}, R^{UA2}, R^{UA7} und R^{UA8} unabhängig aus der Gruppe bestehend aus einer Alkylgruppe und einer Arylgruppe ausgewählt sind und
R^{UA3}, R^{UA4}, jedes R^{UA5} und jedes R^{UA6} unabhängig aus der Gruppe bestehend aus Wasserstoff, einer Alkylgruppe und einer Arylgruppe ausgewählt sind; Y^{UA1} und Y^{UA3} unabhängig für Alkandiylgruppen stehen;
jedes Y^{UA2} unabhängig aus der Gruppe bestehend aus einer Alkandiylgruppe, einer Alkandiyloxyalkandiylgruppe, einer Alkandiyloligo(oxyalkandiyl)-gruppe und einer Alkandiylpoly(oxyalkandiyl)gruppe ausgewählt ist und
α für eine ganze Zahl aus der Gruppe bestehend aus 0, 1, 2 und 3 steht;
die Triamine durch Formel (TA) wiedergegeben werden, 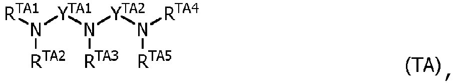 wobei Y^{TA1} und Y^{TA2} unabhängig für Alkandiylgruppen stehen;
R^{TA1}, R^{TA2}, R^{TA3}, R^{TA4} und R^{TA5} unabhängig aus der Gruppe bestehend aus einer Alkylgruppe und einer Arylgruppe ausgewählt sind;
die aromatischen Amine durch Formel (AA) wiedergegeben werden, 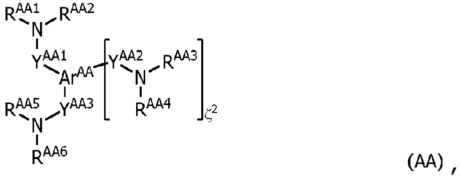 wobei
Ar^{AA} für eine aromatische Gruppe steht;
Y^{AA1}, jedes Y^{AA2} und Y^{AA3} unabhängig für Alkandiylgruppen stehen;
R^{AA1}, R^{AA2}, R^{AA3}, R^{AA4}, R^{AA5}, R^{AA5} und R^{AA6} unabhängig aus der Gruppe bestehend aus einer Alkylgruppe und einer Arylgruppe ausgewählt sind;
ζ² für eine ganze Zahl steht, die aus 1 und 2 ausgewählt ist;
die Diharnstoffoligoamine durch Formel (OA) wiedergegeben werden, 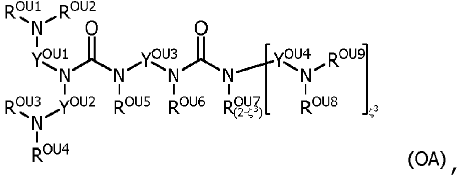 wobei
Y^{OU1}, Y^{OU2}, Y^{OU3} und jedes Y^{OU4} unabhängig für Alkandiylgruppen stehen;
R^{OU5}, R^{OU6} und jedes R^{OU7} unabhängig aus der Gruppe bestehend aus Wasserstoff, einer Alkylgruppe und einer Arylgruppe ausgewählt sind;
R^{OU1}, R^{OU2}, R^{OU3}, R^{OU4}, jedes R^{OU8} und jedes R^{OU9} unabhängig aus der Gruppe bestehend aus einer Alkylgruppe und einer Arylgruppe ausgewählt sind; ζ³ für eine ganze Zahl steht, die aus 1 und 2 ausgewählt ist; und
die cyclischen Aminen durch Formel (CA) wiedergegeben werden, 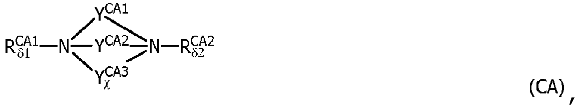 wobei
R^{CA1} und R^{CA2} unabhängig aus der Gruppe bestehend aus einer Alkylgruppe und einer Arylgruppe ausgewählt sind;
Y^{CA1}, Y^{CA2} und Y^{CA3} unabhängig aus der Gruppe bestehend aus Alkandiyl ausgewählt sind;
χ für eine ganze Zahl steht, die aus 0 und 1 ausgewählt ist; und
δ1 für eine ganze Zahl steht, die durch die mathematische Gleichung δ1 = (1-χ) ausgedrückt wird;
δ2 für eine ganze Zahl steht, die durch die mathematische Gleichung δ2 = (1-χ) ausgedrückt wird;
die heterocyclischen Amine durch Formel (HA) wiedergegeben werden, 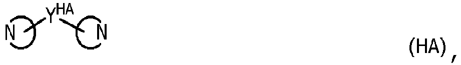 wobei
Y^{HA} aus der Gruppe bestehend aus einer Alkandiylgruppe und einer Alkandiyl-urediyl-alkandiylgruppe ausgewählt ist.

9. Zwischenprodukt, das für die Umsetzung mit einem multifunktionellen Ausgangsstoff gemäß Anspruch 8 zu dem Ureylenadditiv nach einem der Ansprüche 1 bis 7 brauchbar ist, wobei das Zwischenprodukt eine Struktureinheit umfasst, die durch Formel (INT) wiedergegeben wird, 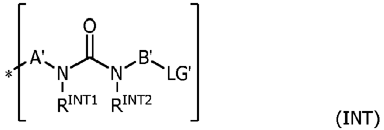 wobei
B' für eine Alkandiylgruppe steht;
LG' für eine durch nukleophile Substitution ersetzbare Abgangsgruppe steht;
R^{INT1'} und R^{INT2'} unabhängig aus der Gruppe bestehend aus Wasserstoff, einer Alkylgruppe und einer Arylgruppe ausgewählt sind;
A' aus der Gruppe bestehend aus einer Oligo(oxy-alkandiyl)gruppe und einer Poly(oxyalkandiyl)-gruppe ausgewählt ist.

10. Verwendung eines Ureylenadditivs nach einem der Ansprüche 1 bis 7 in einer Zusammensetzung zur Abscheidung eines Metalls oder einer Metalllegierung, vorzugsweise in einer Zusammensetzung zur Abscheidung von Kupfer oder einer Kupferlegierung, weiter bevorzugt in einer Zusammensetzung zur elektrolytischen Abscheidung von Kupfer oder einer Kupferlegierung, insbesondere als Einebnungsmittel und/oder Unterdrücker darin.

11. Zusammensetzung zur Abscheidung eines Metalls oder einer Metalllegierung, umfassend mindestens einen Typ von reduzierbaren Metallionen, vorzugsweise Kupferionen, und mindestens ein Ureylenadditiv nach einem der Ansprüche 1 bis 7.

12. Zusammensetzung zur Abscheidung eines Metalls oder einer Metalllegierung nach Anspruch 11, wobei es sich bei dem mindestens einen Typ von reduzierbaren Metallionen um Kupferionen handelt.

13. Verwendung der Metall- oder Metalllegierungszusammensetzung nach Anspruch 11 oder 12 zur Bildung einer Metall- oder Metalllegierungsschicht, vorzugsweise einer Kupfer- oder Kupferlegierungsschicht, auf einer Oberfläche eines Substrats.

14. Verfahren zum Abscheiden einer Metall- oder Metalllegierungsschicht, vorzugsweise einer Kupfer- oder Kupferlegierungsschicht, auf mindestens einer Oberfläche eines Substrats, das die folgenden Verfahrensschritte umfasst:
(i) Bereitstellen des Substrats mit der mindestens einen Oberfläche;
(ii) Inkontaktbringen der mindestens einen Oberfläche des Substrats mit der Zusammensetzung nach einem der Ansprüche 11 oder 12;
(iii) gegebenenfalls Anlegen eines elektrischen Stroms zwischen dem Substrat und mindestens einer Anode;
wodurch die Metall- oder Metalllegierungsschicht, vorzugsweise die Kupfer- oder Kupferlegierungsschicht, auf der mindestens einen Oberfläche des Substrats gebildet wird.

## Revendications

1. Additif de type uréylène comprenant au moins un motif structural représenté par la formule (I) 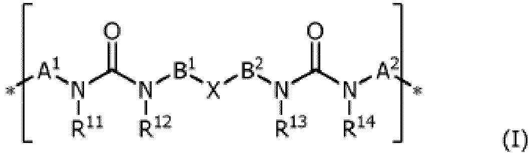 A¹ et A² étant indépendamment choisis dans le groupe constitué par un groupe oligo(oxyalcanediyle) et un groupe poly(oxyalcanediyle) ;
B¹ et B² étant indépendamment des groupes alcanediyle ;
R¹¹, R¹², R¹³ et R¹⁴ étant indépendamment choisis dans le groupe constitué par hydrogène, un groupe alkyle et un groupe aryle ; et
X étant choisi dans le groupe constitué par
- des motifs structuraux représentés par la formule (II) 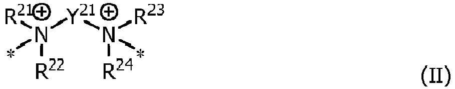 R²¹, R²², R²³ et R²⁴ étant indépendamment choisis dans le groupe constitué par un groupe alkyle et un groupe aryle ; et
Y²¹ étant choisi dans le groupe constitué par un groupe alcanediyle, un groupe alcanediyloxyalcanediyle, un groupe alcanediyloligo(oxyalcanediyle) et un groupe alcanediylpoly(oxyalcanediyle) ;
- des motifs structuraux représentés par la formule (III) 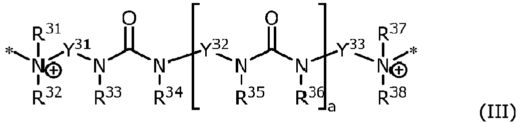 R³¹, R³², R³⁷ et R³⁸ étant indépendamment choisis dans le groupe constitué par un groupe alkyle et un groupe aryle ;
R³³, R³⁴, chaque R³⁵ et chaque R³⁶ étant indépendamment choisis dans le groupe constitué par hydrogène, un groupe alkyle et un groupe aryle ;
Y³¹ et Y³³ étant indépendamment des groupes alcanediyle ;
chaque Y³² étant indépendamment choisi dans le groupe constitué par un groupe alcanediyle, un groupe alcanediyloxyalcanediyle, un groupe alcanediyloligo(oxyalcanediyle) et un groupe alcanediylpoly(oxyalcanediyle) ; et
a étant un entier choisi dans le groupe constitué par 0, 1, 2 et 3 ;
- des motifs structuraux représentés par la formule (IV) 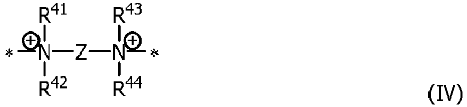 R⁴¹, R⁴², R⁴³ et R⁴⁴ étant indépendamment choisis dans le groupe constitué par un groupe alkyle et un groupe aryle ;
Z étant représenté par l'une des formules suivantes (Z1) à (Z3) 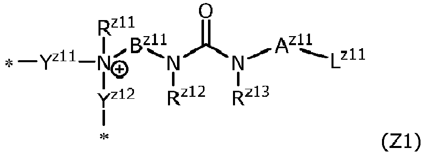 Y^{z11} et Y^{z12} étant indépendamment des groupes alcanediyle ;
A^{z11} étant indépendamment choisi dans le groupe constitué par un groupe oligo(oxyalcanediyle) et un groupe poly(oxyalcanediyle);
B^{z11} étant indépendamment choisi pour être un groupe alcanediyle ;
R^{z11} étant choisi dans le groupe constitué par un groupe alkyle et un groupe aryle ;
R^{z12} et R^{z13} étant indépendamment choisis dans le groupe constitué par hydrogène, un groupe alkyle et un groupe aryle ;
L^{z11} étant choisi dans le groupe constitué par hydrogène, un groupe alkyle et un groupe aryle ; 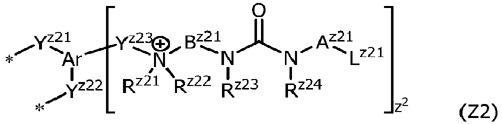 Ar étant un groupe aromatique ;
Y^{z21}, Y^{z22} et chaque Y^{z23} étant indépendamment des groupes alcanediyle ;
chaque A^{z21} étant indépendamment choisi dans le groupe constitué par un groupe oligo(oxyalcanediyle) et un groupe poly(oxyalcanediyle);
chaque B^{z21} étant indépendamment choisi pour être un groupe alcanediyle ;
chaque R^{z21} et chaque R^{z22} étant indépendamment choisis dans le groupe constitué par un groupe alkyle et un groupe aryle ;
chaque R^{z23} et chaque R^{z24} étant indépendamment choisis dans le groupe constitué par hydrogène, un groupe alkyle et un groupe aryle ;
chaque L^{z21} étant indépendamment choisi dans le groupe constitué par hydrogène, un groupe alkyle et un groupe aryle ;
z² étant un entier choisi parmi 1 et 2 ;
et 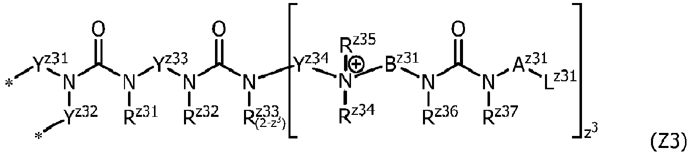 Y^{z31}, Y^{z32}, Y^{z33} et chaque Y^{z34} étant indépendamment des groupes alcanediyle ;
chaque A^{z31} étant indépendamment choisi dans le groupe constitué par un groupe oligo(oxyalcanediyle) et un groupe poly(oxyalcanediyle);
chaque B^{z31} étant indépendamment choisi pour être un groupe alcanediyle ;
R^{z31}, R^{z32}, R^{z33}, chaque R^{z36} et chaque R^{z37} étant indépendamment choisis dans le groupe constitué par hydrogène, un groupe alkyle et un groupe aryle ;
chaque R^{z34} et chaque R^{z35} étant indépendamment choisis dans le groupe constitué par un groupe alkyle et un groupe aryle ;
chaque L^{z31} étant indépendamment choisi dans le groupe constitué par hydrogène, un groupe alkyle et un groupe aryle ;
z³ étant un entier choisi parmi 1 et 2 ;
des motifs structuraux représentés par la formule (V) 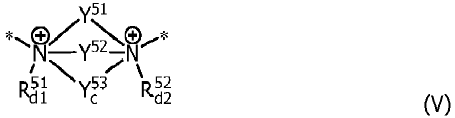 chaque R⁵¹ et chaque R⁵² étant indépendamment choisis dans le groupe constitué par un groupe alkyle et un groupe aryle ;
chacun parmi Y⁵¹, Y⁵² et Y⁵³ étant indépendamment un groupe alcanediyle ;
c étant choisi parmi 0 et 1 ;
d1 étant un entier exprimé par l'équation mathématique d1 = (1 - c) ;
d2 étant un entier exprimé par l'équation mathématique d2 = (1 - c) ; et
et
- des motifs structuraux représentés par la formule (VI) 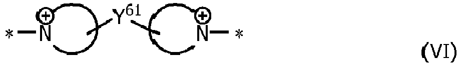 Y⁶¹ étant choisi dans le groupe constitué par un groupe alcanediyle et un groupe alcanediyl-urédiyl-alcanediyle.

2. Additif de type uréylène selon la revendication 1 **caractérisé en ce que** A¹, A², A^{z11}, A^{z21} et A^{z31} sont indépendamment représentés par la formule suivante (A-1) 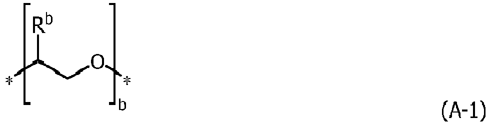 chaque R^{b} étant choisi dans le groupe constitué par hydrogène, un groupe alkyle et un groupe aryle ; préférablement parmi hydrogène et un groupe C1-C4-alkyle ; plus préférablement parmi hydrogène et un groupe méthyle ;
b étant un entier dans la plage de 3 à 100, préférablement de 5 à 75, plus préférablement de 7 à 50.

3. Additif de type uréylène selon l'une quelconque des revendications précédentes **caractérisé en ce que** B¹, B², B^{z11}, B^{z21} et B^{z31} sont indépendamment un groupe C1-C8-alcanediyle, préférablement un groupe C2-C6-alcanediyle, plus préférablement un groupe C2-C4-alcanediyle, ils sont encore plus préférablement choisis dans le groupe constitué par éthane-1,2-diyle et propane-1,3-diyle ; ils sont le plus préférablement éthane-1,2-diyle.

4. Additif de type uréylène selon l'une quelconque des revendications précédentes **caractérisé en ce qu'**il comprend un groupe de coiffage L¹¹ lié à A¹ et/ou un groupe de coiffage L¹² lié à A², les groupes de coiffage L¹¹ et/ou L¹² étant indépendamment choisis dans le groupe constitué par hydrogène, un groupe alkyle et un groupe aryle ; L¹¹ et/ou L¹² étant préférablement indépendamment des groupes alkyle, plus préférablement des groupes C1-C4-alkyle, encore plus préférablement des groupes C1-C2-alkyle, même encore plus préférablement des groupes méthyle.

5. Additif de type uréylène selon l'une quelconque des revendications précédentes, **caractérisé en ce que** R¹¹, R¹², R¹³, R¹⁴, R³³, R³⁴, R³⁵, R³⁶, R^{z12}, R^{z13}, R^{z23}, R^{z24}, R^{z31}, R^{z32}, R^{z33}, R^{z36} et R^{z37} sont indépendamment choisis dans le groupe constitué par hydrogène et un groupe C1-C4-alkyle ; plus préférablement ils sont hydrogène.

6. Additif de type uréylène selon l'une quelconque des revendications précédentes, **caractérisé en ce que** R²¹, R²², R²³, R²⁴, R³¹, R³², R³⁷, R³⁸, R⁴¹, R⁴², R⁴³, R⁴⁴, R⁵¹, R⁵², R^{z11}, R^{z21}, R^{z22}, R^{z34} et R^{z35} sont choisis indépendamment les uns des autres sont indépendamment des groupes C1-C4-alkyle ; plus préférablement des groupes C1-C2-alkyle ; le plus préférablement ils sont des groupes méthyle.

7. Additif de type uréylène selon l'une quelconque des revendications précédentes, **caractérisé en ce que** X est choisi parmi des motifs structuraux représentés par la formule (II) et des motifs structuraux représentés par la formule (III), préférablement X est un motif structural représenté par la formule (III).

8. Procédé pour la préparation d'un additif de type uréylène par la mise en réaction d'au moins un isocyanate représenté par la formule (ISO) 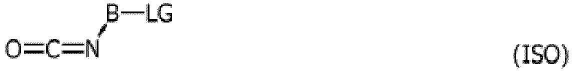 B étant un groupe alcanediyle ; et
LG étant un groupe partant approprié pour être remplacé par substitution nucléophile ; avec
au moins une monoamine comprenant un motif structural représenté par la formule (MA) 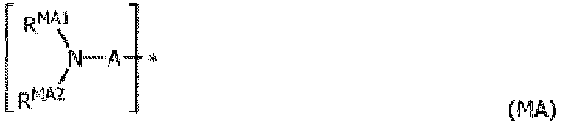 A étant choisi dans le groupe constitué par un groupe oligo(oxyalcanediyle) et un groupe poly(oxyalcanediyle) ; et
chaque R^{MA1} et R^{MA2} étant choisis dans le groupe constitué par hydrogène, un groupe alkyle et un groupe aryle ;
et formant ainsi au moins un intermédiaire ;
et ledit au moins un intermédiaire étant mis à réagir avec au moins un produit de départ multifonctionnel choisi dans le groupe constitué par des diamines, des uréeamines, des triamines, des amines aromatiques, des diuréeoligoamines, des amines cycliques et des amines hétérocycliques, les diamines étant représentées par la formule (DA) 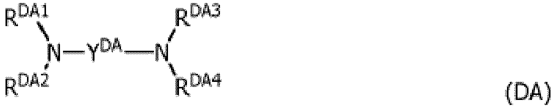 R^{DA1}, R^{DA2}, R^{DA3} et R^{DA4} étant indépendamment choisis dans le groupe constitué par un groupe alkyle et un groupe aryle ; et
Y^{DA} étant choisi dans le groupe constitué par un groupe alcanediyle, un groupe alcanediyloxyalcanediyle, un groupe alcanediyloligo(oxyalcanediyle), un groupe alcanediylpoly(oxyalcanediyle) ;
les uréeamines étant représentées par la formule (UA) 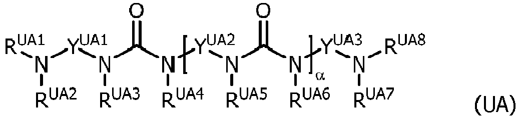 R^{UA1}, R^{UA2}, R^{UA7} et R^{UA8} étant indépendamment choisis dans le groupe constitué par un groupe alkyle et un groupe aryle ; et
R^{UA3}, R^{UA4}, chaque R^{UA5} et chaque R^{UA6} étant indépendamment choisis dans le groupe constitué par hydrogène, un groupe alkyle et un groupe aryle ;
Y^{UA1} et Y^{UA3} étant indépendamment des groupes alcanediyle ;
chaque Y^{UA2} étant indépendamment choisi dans le groupe constitué par un groupe alcanediyle, un groupe alcanediyloxyalcanediyle, un groupe alcanediyloligo(oxyalcanediyle) et un groupe alcanediylpoly(oxyalcanediyle) ; et
α étant un entier choisi dans le groupe constitué par 0, 1, 2 et 3 ;
les triamines étant représentées par la formule (TA) 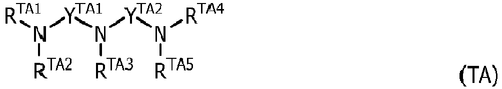 Y^{TA1} et Y^{TA2} étant indépendamment des groupes alcanediyle ;
R^{TA1}, R^{TA2}, R^{TA3}, R^{TA4} et R^{TA5} étant indépendamment choisis dans le groupe constitué par un groupe alkyle et un groupe aryle ;
les amines aromatiques étant représentées par la formule (AA) 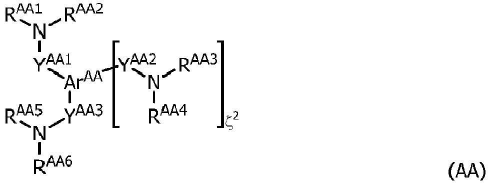 Ar^{AA} étant un groupe aromatique ; Y^{AA1}, chaque Y^{AA2} et Y^{AA3} étant indépendamment des groupes alcanediyle ;
R^{AA1}, R^{AA2}, R^{AA3}, R^{AA4}, R^{AA5}, R^{AA5} et R^{AA6} étant indépendamment choisis dans le groupe constitué par un groupe alkyle et un groupe aryle ;
ζ² étant un entier choisi parmi 1 et 2 ;
les diuréeoligoamines étant représentées par la formule (OA) 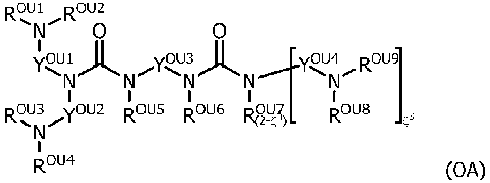 Y^{OU1}, Y^{OU2}, Y^{OU3} et chaque Y^{OU4} étant indépendamment des groupes alcanediyle ;
R^{OU5}, R^{OU6} et chaque R^{OU7} étant indépendamment choisis dans le groupe constitué par hydrogène, un groupe alkyle et un groupe aryle ;
R^{OU1}, R^{OU2}, R^{OU3}, R^{OU4}, chaque R^{OU8}, et chaque R^{OU9} étant indépendamment choisis dans le groupe constitué par un groupe alkyle et un groupe aryle ;
ζ³ étant un entier choisi parmi 1 et 2 ; et
les amines cycliques étant représentées par la formule (CA) 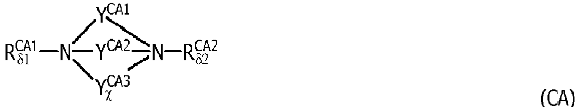 R^{CA1} et R^{CA2} étant indépendamment choisis dans le groupe constitué par un groupe alkyle et un groupe aryle ;
Y^{CA1}, Y^{CA2} et Y^{CA3} étant indépendamment choisis dans le groupe constitué par alcanediyle ;
χ étant un entier choisi parmi 0 et 1 ; et
δ1 étant un entier exprimé par l'équation mathématique δ1 = (1 - χ) ;
δ2 étant un entier exprimé par l'équation mathématique δ2 = (1 - χ) ;
les amines hétérocycliques étant représentées par la formule (HA) 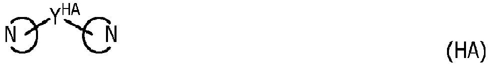 Y^{HA} étant choisi dans le groupe constitué par un groupe alcanediyle et un groupe alcanediyl-urédiyl-alcanediyle.

9. Intermédiaire utile pour être mis à réagir avec un produit de départ multifonctionnel tel que défini dans la revendication 8 pour former un additif de type uréylène selon l'une quelconque des revendications 1 à 7, l'intermédiaire comprenant un motif structural représenté par la formule (INT) 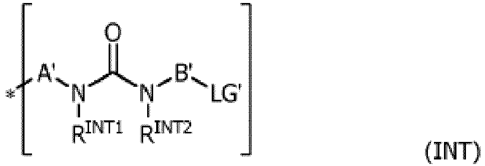 B' étant un groupe alcanediyle ;
LG' étant un groupe partant approprié pour être remplacé par substitution nucléophile ;
R^{INT1'} et R^{INT2'} étant choisis indépendamment dans le groupe constitué par hydrogène, un groupe alkyle et un groupe aryle ;
A' étant choisi dans le groupe constitué par un groupe oligo(oxyalcanediyle) et un groupe poly(oxyalcanediyle) .

10. Utilisation d'un additif de type uréylène selon l'une quelconque des revendications 1 à 7 dans une composition de dépôt de métal ou d'alliage métallique, préférablement dans une composition de dépôt de cuivre ou d'alliage de cuivre, plus préférablement dans une composition de dépôt électrolytique de cuivre ou d'alliage de cuivre, en particulier en tant qu'agent niveleur et/ou agent suppresseur dans celle-ci.

11. Composition de dépôt de métal ou d'alliage métallique comprenant au moins un type d'ions métalliques réductibles, préférablement des ions cuivre, et au moins un additif de type uréylène selon l'une quelconque des revendications 1 à 7.

12. Composition de dépôt de métal ou d'alliage métallique selon la revendication 11, l'au moins un type d'ions métalliques réductibles étant des ions cuivre.

13. Utilisation de la composition de métal ou d'alliage métallique selon l'une quelconque des revendications 11 et 12 pour former une couche de métal ou d'alliage métallique, préférablement une couche de cuivre ou d'alliage de cuivre, sur une surface d'un substrat.

14. Procédé pour le dépôt d'une couche de métal ou d'alliage métallique, préférablement d'une couche de cuivre ou d'alliage de cuivre, sur au moins une surface d'un substrat comprenant les étapes de procédé suivantes
(i) mise à disposition du substrat possédant ladite au moins une surface
(ii) mise en contact de ladite au moins une surface du substrat avec la composition selon l'une quelconque des revendications 11 et 12 ;
(iii) éventuellement, application d'un courant électrique entre le substrat et au moins une anode ; et formant ainsi la couche de métal ou d'alliage métallique, préférablement la couche de cuivre ou d'alliage de cuivre, sur ladite au moins une surface du substrat.
